(19) Europäisches Patentamt / European Patent Office / Office européen des brevets

(11) **EP 3 394 015 B1**

(12) **FASCICULE DE BREVET EUROPEEN**

(45) Date de publication et mention
de la délivrance du brevet:
**08.11.2023   Bulletin 2023/45**

(21) Numéro de dépôt: **16809096.7**

(22) Date de dépôt: **14.12.2016**

(51) Classification Internationale des Brevets (IPC):
**C07C 17/23** $^{(2006.01)}$     **C07C 17/386** $^{(2006.01)}$
**C07C 21/18** $^{(2006.01)}$

(52) Classification Coopérative des Brevets (CPC):
(C-Sets disponibles)
**C07C 17/386; C07C 17/25;** C09K 5/045;
C09K 2205/126                                            (Cont.)

(86) Numéro de dépôt international:
**PCT/EP2016/080952**

(87) Numéro de publication internationale:
**WO 2017/108524 (29.06.2017 Gazette 2017/26)**

(54) **PROCEDE DE PRODUCTION ET DE PURIFICATION DU 2,3,3,3-TETRAFLUORO-1-PROPENE**

VERFAHREN ZUR HERSTELLUNG UND REINIGUNG VON 2,3,3,3-TETRAFLUOR-1-PROPEN

METHOD FOR PRODUCING AND PURIFYING 2,3,3,3-TETRAFLUORO-1-PROPENE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité:  **23.12.2015  FR 1563169**

(43) Date de publication de la demande:
**31.10.2018   Bulletin 2018/44**

(73) Titulaire: **Arkema France
92700 Colombes (FR)**

(72) Inventeurs:
• **BABA-AHMED, Abdelatif
69190 Saint-fons (FR)**
• **COLLIER, Bertrand
69230 Saint-genis-laval (FR)**
• **DEUR-BERT, Dominique
69390 Charly (FR)**
• **EMERY, Irène
69340 Francheville (FR)**
• **WENDLINGER, Laurent
69510 Soucieu En Jarrest (FR)**

(74) Mandataire: **Arkema Patent
Arkema France
DRD-DPI
420, rue d'Estienne d'Orves
92705 Colombes Cedex (FR)**

(56) Documents cités:
**WO-A1-2012/011609     WO-A2-2008/030440**

**(Cont. page suivante)**

(52) Classification Coopérative des Brevets (CPC):
(Cont.)

C-Sets
**C07C 17/25, C07C 21/18;**
**C07C 17/386, C07C 21/18**

## Description

### Domaine technique de l'invention

L'invention se rapporte à un procédé de purification du 2,3,3,3-tetrafluoro-1-propène. En outre, l'invention se rapporte également à un procédé de production et de purification du 2,3,3,3-tetrafluoro-1-propène.

### Arrière-plan technologique de l'invention

Les hydrofluorocarbures (HFC) et en particulier les hydrofluorooléfines (HFOs), telles que le 2,3,3,3-tétrafluoro-1-propène (HFO-1234yf) sont des composés connus pour leurs propriétés de réfrigérants et fluides caloporteurs, extinctrices, propulseurs, agents moussants, agents gonflants, diélectriques gazeux, milieu de polymérisation ou monomère, fluides supports, agents pour abrasifs, agents de séchage et fluides pour unité de production d'énergie. Les HFO ont été identifiés comme des alternatives souhaitables au HCFC du fait de leurs faibles valeurs d'ODP (Ozone Depletion Potential ou potentiel d'appauvrissement de la couche d'ozone) et de GWP (Global Warming Potential ou potentiel de réchauffement climatique).

La plupart des procédés de fabrication des hydrofluorooléfines font appel à une réaction de fluoration et/ou déshydrohalogénation. Ce type de réaction est effectué en phase gazeuse et génère des impuretés qu'il faut par conséquent éliminer pour obtenir le composé désiré dans un degré de pureté suffisant pour les applications visées.

Par exemple, dans le cadre de la production de 2,3,3,3-tétrafluoro-1-propène (HFO-1234yf), la présence d'impuretés telle que le trans-1,3,3,3-tetrafluoro-1-propene (1234ze-E) est observée. Des produits secondaires tels que par exemple le 1,1,1,2,2-pentafluoropropène sont également formés. La purification de ce type de mélange réactionnel peut être effectuée par exemple par distillation. Cependant lorsque les composés à purifier ont des points d'ébullition trop proches ou lorsque ceux-ci forment des compositions azéotropes ou quasi-azéotropes, la distillation n'est pas un procédé efficace. Des procédés de distillation extractive ont ainsi été décrits.

On connaît par WO 03/068716 un procédé de récupération de pentafluoroéthane à partir d'un mélange comprenant du pentafluoroéthane et du chloropentafluoroéthane par distillation en présence d'hexafluoropropène.

On connaît aussi par WO 98/19982 un procédé de purification du 1,1-difluoroéthane par distillation extractive. Le procédé consiste à mettre en contact un agent d'extraction avec un mélange de 1,1-difluoroéthane et de chlorure de vinyle. L'agent d'extraction est choisi parmi les hydrocarbures, les alcools, les chlorocarbures ayant un point d'ébullition compris entre 10°C et 120°C. Comme mentionné par WO 98/19982, la sélection de l'agent d'extraction peut s'avérer complexe en fonction des produits à séparer. On connaît aussi par WO2012/011609 un procédé de purification du 2,3,3,3-tétrafluoro-1-propène par distillation extractive d'une compositition comprenant du 2,3,3,3-tétrafluoro-1-propène.

### Résumé de l'invention

La présente invention permet la purification du 2,3,3,3-tetrafluoro-1-propène. La présente invention permet en particulier l'obtention d'un courant comprenant du 2,3,3,3-tetrafluoro-1-propène et du 1,1,1,2,2-pentafluoropropane ayant une faible teneur en trans-1,3,3,3-tetrafluoro-1-propene (1234ze-E). La présente invention permet ainsi la production de 2,3,3,3-tetrafluoro-1-propène avec une pureté améliorée.

Selon un premier aspect, la présente invention fournit un procédé de purification du 2,3,3,3-tétrafluoro-1-propène (1234yf) à partir d'une première composition comprenant du 2,3,3,3-tétrafluoro-1-propène, 1,1,1,2,2-pentafluoropropane (245cb) et trans-1,3,3,3-tetrafluoro-1-propene (1234ze-E), ledit procédé comprenant les étapes de:

a) mise en contact de ladite première composition avec au moins un agent d'extraction organique pour former une seconde composition ;
b) distillation extractive de ladite seconde composition pour former :

i) une troisième composition comprenant ledit agent d'extraction organique et trans-1,3,3,3-tetrafluoro-1-propène (1234ze-E); et
ii) un courant comprenant le 2,3,3,3-tétrafluoro-1-propène et le 1,1,1,2,2-pentafluoropropane (245cb),

c) récupération et séparation de ladite troisième composition pour former un courant comprenant ledit agent d'extraction organique et un courant comprenant le trans-1,3,3,3-tetrafluoro-1-propène (1234ze-E).

De préférence, la séparation de l'étape c) est effectuée par distillation. De préférence, le courant comprenant ledit agent d'extraction organique est recyclé à l'étape a). De préférence le courant comprenant le trans-1,3,3,3-tetrafluoro-1-propène est purifié ou détruit par incinération.

De préférence, le courant ii) comprenant le 2,3,3,3-tétrafluoro-1-propène et le 1,1,1,2,2-pentafluoropropane (245cb) formé à l'étape b) est récupéré. Le courant peut être purifié pour séparer le 2,3,3,3-tétrafluoro-1-propène et le 1,1,1,2,2-pentafluoropropane (245cb). Ainsi, selon un mode de réalisation préféré, le procédé comprend également une étape de récupération dudit courant comprenant le 2,3,3,3-tétrafluoro-1-propène et le 1,1,1,2,2-pentafluoropropane (245cb) obtenu à l'étape b), et de distillation de celui-ci pour former un courant A comprenant 2,3,3,3-tétrafluoro-1-propène et un courant B comprenant 1,1,1,2,2-pentafluoropropane (245cb). Le courant B comprenant 1,1,1,2,2-pentafluoropropane (245cb) peut être recyclé à l'étape A) du procédé de production et de purification du 2,3,3,3-tétrafluoro-1-propène décrit ci-dessous. Le courant A comprenant 2,3,3,3-tétrafluoro-1-propène peut être purifié pour atteindre un degré de pureté respectant les spécifications commerciales, i.e. dépourvu de ou présentant une faible teneur en 1,1,1,2,2-pentafluoropropane ou de trans-1,3,3,3-tetrafluoro-1-propène.

Selon un mode de réalisation préféré, ladite première composition comprend également au moins un des composés sélectionnés parmi le groupe consistant en chlorométhane (40), 1,1-difluoroéthane (152a), 1,1,1,2-tétrafluo-roethane (134a), chloropentafluoroéthane (115), trans-1,2,3,3,3-pentafluoropropene (1225ye-E), cis-1,2,3,3,3-pentafluoropropene (1225ye-Z) et 3,3,3-trifluoropropene (1243zf).

Selon un mode de réalisation préféré, lesdits au moins un des composés sélectionnés parmi le groupe consistant en chlorométhane (40), 1,1-difluoroéthane (152a), 1,1,1,2-tétrafluoroethane (134a), chloropentafluoroéthane (115), trans-1,2,3,3,3-pentafluoropropene (1225ye-E), cis-1,2,3,3,3-pentafluoropropene (1225ye-Z), 3,3,3-trifluoropropene (1243zf) sont également contenus dans lesdites seconde et troisième compositions ; l'étape c) du présent procédé étant la récupération et la séparation de ladite troisième composition entre d'une part un courant comprenant ledit agent d'extraction organique et d'autre part un courant comprenant le trans-1,3,3,3-tetrafluoro-1-propene (1234ze-E) et ledit au moins un des composés sélectionnés parmi le groupe consistant en chlorométhane (40), 1,1-difluoroéthane (152a), 1,1,1,2-tétrafluoroethane (134a), chloropentafluoroéthane (115), trans-1,2,3,3,3-pentafluoropropene (1225ye-E), cis-1,2,3,3,3-pentafluoropropene (1225ye-Z), 3,3,3-trifluoropropene (1243zf).

De préférence, ledit agent d'extraction organique peut avoir un point d'ébullition compris entre 10 et 150°C.

Selon un mode de réalisation préféré, ledit agent d'extraction organique est un solvant choisi parmi le groupe consistant en hydrohalocarbure, alcool, cétone, amine, ester, éther, aldéhyde, nitrile, carbonate, thioalkyle, amide, hétérocycle; ou l'agent d'extraction organique est triethylfluorosilane. De préférence, l'agent d'extraction organique est choisi parmi le groupe consistant en amine, éther, ester, aldéhyde, cétone, alcool et hétérocycle.

Selon un mode de réalisation préféré ledit agent d'extraction organique a un facteur de séparation $S_{1,2}$ supérieur ou égal à 1,1, ledit facteur de séparation étant calculé par la formule $S_{1,2} = (\gamma_{1,S}*P1)/(\gamma_{2,S}*P2)$ dans laquelle

$\gamma_{1,S}$ représente le coefficient d'activité du 2,3,3,3-tétrafluoro-1-propène dans ledit agent d'extraction organique à dilution infinie,
P1 représente la pression de vapeur saturante du 2,3,3,3-tétrafluoro-1-propène,
$\gamma_{2,S}$ représente le coefficient d'activité du trans-1,3,3,3-tetrafluoro-1-propene (1234ze-E) dans ledit agent d'extraction organique à dilution infinie,
P2 représente la pression de vapeur saturante du trans-1,3,3,3-tetrafluoro-1-propene (1234ze-E) ;

avantageusement, le facteur de séparation est supérieur ou égal à 1,2, de préférence supérieur ou égal à 1,4, plus préférentiellement supérieur ou égal à 1,6, en particulier supérieur ou égal à 1,8.

Selon un mode de réalisation préféré, ledit agent d'extraction organique a une capacité d'absorption $C_{2,S}$ supérieure ou égale à 0,20, ladite capacité d'absorption étant calculé par la formule $C_{2,S} = 1/(\gamma_{2,S})$ dans laquelle $\gamma_{2,S}$ représente le coefficient d'activité dudit au moins un des composés consistant en trans-1,3,3,3-tetrafluoro-1-propene (1234ze-E) dans ledit agent d'extraction organique à dilution infinie, de préférence $\gamma_{2,S}$ représente le coefficient d'activité dudit au moins un des composés consistant en trans-1,3,3,3-tetrafluoro-1-propene (1234ze-E), dans ledit agent d'extraction organique à dilution infinie ;

avantageusement, la capacité d'absorption $C_{2,S}$ est supérieure ou égale à 0,40, de préférence supérieure ou égale à 0,60, plus préférentiellement supérieure ou égale à 0,80, en particulier supérieure ou égale à 1,0.

Selon un mode de réalisation préféré ledit agent d'extraction organique peut également avoir un facteur de séparation $S_{1,2}$ supérieur ou égal à 1,1, ledit facteur de séparation étant calculé par la formule $S_{1,2} = (\gamma_{1,S}*P1)/(\gamma_{2,S}*P2)$ dans laquelle

$\gamma_{1,S}$ représente le coefficient d'activité du 1,1,1,2,2-pentafluoropropane (245cb) dans ledit agent d'extraction organique à dilution infinie,
P1 représente la pression de vapeur saturante du 1,1,1,2,2-pentafluoropropane (245cb), $\gamma_{2,S}$ représente le coefficient d'activité du trans-1,3,3,3-tetrafluoro-1-propene (1234ze-E) dans ledit agent d'extraction organique à dilution infinie,
P2 représente la pression de vapeur saturante du trans-1,3,3,3-tetrafluoro-1-propene (1234ze-E) ;

avantageusement, le facteur de séparation est supérieur ou égal à 1,2, de préférence supérieur ou égal à 1,4, plus préférentiellement supérieur ou égal à 1,6, en particulier supérieur ou égal à 1,8, plus particulièrement supérieur ou égal à 2,0.

Selon un mode de réalisation préféré, ladite première composition peut comprendre 2,3,3,3-tetrafluoro-1-propène, 1,1,1,2,2-pentafluoropropane (245cb), trans-1,3,3,3-tetrafluoro-1-propene (1234ze-E) et optionnellement ou non au moins un des composés sélectionnés parmi le groupe consistant en chlorométhane (40), 1,1-difluoroéthane (152a), 1,1,1,2-tetrafluoroethane (134a), chloropentafluoroéthane (115), trans-1,2,3,3,3-pentafluoropropene (1225ye-E), cis-1,2,3,3,3-pentafluoropropene (1225ye-Z), 3,3,3-trifluoropropene (1243zf).

Selon un mode de réalisation préféré, ledit agent d'extraction organique est choisi parmi le groupe consistant en éthylamine, isopropylamine, diethylether, n-propylamine, diethylamine, propanone, methylacetate, butanone, diethoxymethane, isopropylacetate, 3-pentylamine, 2-methoxyethanamine, tert-butylacetate, dioxane, 1,1-diethoxyethane, trimethoxymethane, n-pentylamine, 1,3-dioxane, sec-butylacetate, 1,2-diaminoethane, 1-methoxy-2-propanol, 1,2-propanediamine, n-butylacetate, 2-methoxy-1-propanol, hexanal. De préférence, ledit agent d'extraction organique est choisi parmi le groupe consistant en éthylamine, isopropylamine, diethylether, n-propylamine, diethylamine, diethoxymethane, isopropylacetate, 3-pentylamine, 2-methoxyethanamine, tert-butylacetate, dioxane, 1,1-diethoxyethane, trimethoxymethane, n-pentylamine, 1,3-dioxane, sec-butylacetate, 1,2-diaminoethane, 1-methoxy-2-propanol, 1,2-propanediamine, n-butylacetate, 2-methoxy-1-propanol, hexanal.

Selon un mode de réalisation préféré, le courant comprenant le 2,3,3,3-tetrafluoro-1-propène et 1,1,1,2,2-pentafluoropropane (245cb) formé à l'étape b) est récupéré et est dépourvu de trans-1,3,3,3-tetrafluoro-1-propene (1234ze-E).

Selon un mode de réalisation préféré, le procédé comprend préalablement à l'étape a) les étapes :

i') mise en oeuvre d'une composition comprenant 2,3,3,3-tetrafluoro-1-propène, des impuretés ayant un point d'ébullition inférieur au point d'ébullition du 2,3,3,3-tetrafluoro-1-propène, 1,1,1,2,2-pentafluoropropane (245cb), et trans-1,3,3,3-tetrafluoro-1-propene (1234ze-E), optionnellement ou non au moins un des composés sélectionnés parmi le groupe consistant en chlorométhane (40), 1,1-difluoroéthane (152a), 1,1,1,2-tetrafluoroethane (134a), chloropentafluoroéthane (115), trans-1,2,3,3,3-pentafluoropropene (1225ye-E), cis-1,2,3,3,3-pentafluoropropene (1225ye-Z), 3,3,3-trifluoropropene (1243zf), et optionnellement ou non des impuretés lourdes ;

ii') distillation de ladite composition de l'étape i) pour éliminer en tête de colonne des impuretés ayant un point d'ébullition inférieur au point d'ébullition du 2,3,3,3-tetrafluoro-1-propène et former un premier courant comprenant 2,3,3,3-tetrafluoro-1-propène, 1,1,1,2,2-pentafluoropropane (245cb), et trans-1,3,3,3-tetrafluoro-1-propene (1234ze-E), optionnellement ou non au moins un des composés sélectionnés parmi le groupe consistant en chlorométhane (40), 1,1-difluoroéthane (152a), 1,1,1,2-tetrafluoroethane (134a), chloropentafluoroéthane (115), trans-1,2,3,3,3-pentafluoropropene (1225ye-E), cis-1,2,3,3,3-pentafluoropropene (1225ye-Z), 3,3,3-trifluoropropene (1243zf), et optionnellement ou non des impuretés lourdes, récupéré en bas de colonne de distillation ;

iii') optionnellement ou non, distillation dudit premier courant récupéré en bas de colonne de distillation à l'étape ii') pour récupérer en tête de colonne un second courant comprenant du 2,3,3,3-tetrafluoro-1-propène, 1,1,1,2,2-pentafluoropropane (245cb), et trans-1,3,3,3-tetrafluoro-1-propene (1234ze-E), optionnellement ou non au moins un des composés sélectionnés parmi le groupe consistant en chlorométhane (40), 1,1-difluoroéthane (152a), 1,1,1,2-tetrafluoroethane (134a), chloropentafluoroéthane (115), trans-1,2,3,3,3-pentafluoropropene (1225ye-E), cis-1,2,3,3,3-pentafluoropropene (1225ye-Z), 3,3,3-trifluoropropene (1243zf), et en bas de colonne de distillation un courant comprenant les impuretés lourdes ;

ledit premier courant récupéré à l'étape ii') ou ledit second courant récupéré à l'étape iii') correspondent à ladite première composition mise en oeuvre à l'étape a).

Les impuretés lourdes peuvent contenir par exemple 1,1,1,3,3,3-hexafluoropropane (236fa), 1,1,1,2,3,3-hexafluoropropane (236ea), 1,1,1,2,3,3,3-heptafluoropropane (227ca), cis-1,3,3,3-tetrafluoro-1-propene (1234ze-Z), des dimères ou des trimères issus de l'un des composés présents dans la composition ou le courant considéré.

Selon un second aspect, l'invention fournit un procédé de production et de purification du 2,3,3,3-tetrafluoro-1-propène comprenant les étapes de :

A) fluoration et/ou en présence d'un catalyseur d'un composé de formule $CX(Y)_2$-$CX(Y)_m$-$CH_mXY$ (I) dans laquelle X et Y représentent indépendamment H, F, ou Cl et m = 0 ou 1 ; et/ou fluoration catalytique en présence d'un catalyseur d'un composé de formule $(CX_nY_{3-n})CH_pX_{1-p}CH_mX_{2-m}$ (II) dans lequel X est indépendamment les uns des autres Cl, F, I ou Br ; Y est indépendamment les uns des autres H, Cl, F, I ou Br ; n est 1, 2 ou 3 ; et m est 0, 1 ou 2 ; et p est 0 ou 1 ;

B) récupération d'un courant comprenant du 2,3,3,3-tetrafluoro-1-propène, 1,1,1,2,2-pentafluoropropane (245cb), trans-1,3,3,3-tetrafluoro-1-propene (1234ze-E), et optionnellement ou non au moins un des composés sélectionnés parmi le groupe consistant en chlorométhane (40), 1,1-difluoroéthane (152a), 1,1,1,2-tetrafluoroethane (134a), chloropentafluoroéthane (115), trans-1,2,3,3,3-pentafluoropropene (1225ye-E), cis-1,2,3,3,3-pentafluoropropene (1225ye-Z), 3,3,3-trifluoropropene (1243zf) ;

C) mise en oeuvre du procédé de purification du 2,3,3,3-tetrafluoro-1-propène selon la présente invention à partir du courant récupéré à l'étape B).

Selon un autre aspect, la présente invention fournit une composition comprenant du 2,3,3,3-tetrafluoropropène, 1,1,1,2,2-pentafluoropropane (245cb), trans-1,3,3,3-tetrafluoro-1-propene (1234ze-E) et un agent d'extraction organique ayant un facteur de séparation $S_{1,2}$ supérieur ou égal à 1,60, ledit facteur de séparation étant calculé par la formule $S_{1,2}$ = $(\gamma_{1,3}*P1)/(\gamma_{2,3}*P2)$ dans laquelle $\gamma_{1,S}$ représente le coefficient d'activité du 2,3,3,3-tetrafluoropropène dans ledit agent d'extraction organique à dilution infinie, P1 représente la pression de vapeur saturante du 2,3,3,3-tetrafluoropropène, $\gamma_{2,S}$ représente le coefficient d'activité du trans-1,3,3,3-tetrafluoro-1-propene (1234ze-E) dans ledit agent d'extraction organique à dilution infinie, P2 représente la pression de vapeur saturante du trans-1,3,3,3-tetrafluoro-1-propene (1234ze-E) ; et ledit agent d'extraction organique ayant un facteur de séparation $S_{1,2}$ supérieur ou égal à 1,60, ledit facteur de séparation étant calculé par la formule $S_{1,2}$ = $(\gamma_{1,S}*P1)/(\gamma_{2,S}*P2)$ dans laquelle $\gamma_{1,S}$ représente le coefficient d'activité du 1,1,1,2,2-pentafluoropropane (245cb) dans ledit agent d'extraction organique à dilution infinie, P1 représente la pression de vapeur saturante du 1,1,1,2,2-pentafluoropropane (245cb), $\gamma_{2,S}$ représente le coefficient d'activité du trans-1,3,3,3-tetrafluoro-1-propene (1234ze-E) dans ledit agent d'extraction organique à dilution infinie, P2 représente la pression de vapeur saturante du trans-1,3,3,3-tetrafluoro-1-propene (1234ze-E) de préférence ledit agent d'extraction organique a une capacité d'absorption $C_{2,S}$ supérieure ou égale à 0,60, ladite capacité d'absorption étant calculé par la formule $C_{2,S}$ = $1/(\gamma_{2,S})$ dans laquelle $\gamma_{2,S}$ représente le coefficient d'activité du trans-1,3,3,3-tetrafluoro-1-propene (1234ze-E) dans ledit agent d'extraction organique à dilution infinie. Selon un mode de réalisation préféré, ledit agent d'extraction organique est choisi parmi le groupe consistant en éthylamine, isopropylamine, diethylether, n-propylamine, diethylamine, propanone, methylacetate, butanone, diethoxymethane, isopropylacetate, 3-pentylamine, 2-methoxyethanamine, tert-butylacetate, dioxane, 1,1-diethoxyethane, trimethoxymethane, n-pentylamine, 1,3-dioxane, sec-butylacetate, 1,2-diaminoethane, 1-methoxy-2-propanol, 1,2-propanediamine, n-butylacetate, 2-methoxy-1-propanol, hexanal. De préférence, ledit agent d'extraction organique est choisi parmi le groupe consistant en éthylamine, isopropylamine, diethylether, n-propylamine, diethylamine, diethoxymethane, isopropylacetate, 3-pentylamine, 2-methoxyethanamine, tert-butylacetate, dioxane, 1,1-diethoxyethane, trimethoxymethane, n-pentylamine, 1,3-dioxane, sec-butylacetate, 1,2-diaminoethane, 1-methoxy-2-propanol, 1,2-propanediamine, n-butylacetate, 2-methoxy-1-propanol, hexanal. De préférence, ladite composition selon cet autre aspect de l'invention est dépourvue de HF.

## Brève description des figures

Les figures 1a-c représentent schématiquement un dispositif mettant en oeuvre un procédé de purification du 2,3,3,3-tetrafluoro-1-propène selon un mode de réalisation particulier de la présente invention.

La figure 2 représente schématiquement un dispositif mettant en oeuvre un procédé de production du 2,3,3,3-tetrafluoro-1-propène selon un mode de réalisation particulier de la présente invention.

## Description détaillée de l'invention

Le terme « hydrocarbure » tel qu'utilisé ici se réfère à des composés linéaires ou branchés d'alcane en $C_1$-$C_{20}$, cycloalcane en $C_3$-$C_{20}$, alcène en $C_2$-$C_{20}$, cycloalcène en $C_3$-$C_{20}$, arène en $C_6$-$C_{18}$. Par exemple, le terme alcane se réfère à des composés de formule $C_nH_{2n+2}$ dans lequel n est compris entre 1 et 20. Le terme alcane en $C_1$-$C_{20}$ englobe par exemple le pentane, hexane, heptane, octane, nonane, décane ou des isomères de ceux-ci. Le terme alcène en $C_2$-$C_{20}$ se réfère à des composés hydrocarbonés comprenant une ou plusieurs doubles liaisons carbone-carbone et comprenant de 2 à 20 atomes de carbone. Le terme cycloalcane en $C_3$-$C_{20}$ se réfère à un cycle hydrocarboné saturé comprenant de 3 à 20 atomes de carbone. Le terme aryle en $C_6$-$C_{18}$ se réfère à des composés hydrocarbonés cycliques et aromatiques comprenant de 6 à 18 atomes de carbone. Le terme cycloalcène en $C_3$-$C_{20}$ se réfère à des composés hydrocarbonés cycliques comprenant de 3 à 20 atomes de carbone et comprenant une ou plusieurs doubles liaisons carbone-carbone.

Le terme « alkyle » désigne un radical monovalent issu d'un alcane, linéaire ou branché, comprenant de 1 à 20 atomes de carbone. Le terme « cycloalkyle » désigne un radical monovalent issu d'un cycloalcane comprenant de 3 à 20 atomes de carbone. Le terme « aryle » désigne un radical monovalent issu d'un arène comprenant de 6 à 18 atomes de carbone. Le terme « alkényle » désigne un radical monovalent de 2 à 20 atomes de carbone et au moins une double liaison carbone-carbone. Le terme « alkynyle » désigne un radical monovalent de 2 à 20 atomes de carbone et au moins une triple liaison carbone-carbone. Le terme « halogène » se réfère à un groupement -F, -Cl, -Br ou -I. Le terme « cycloalkényle » se réfère à un radical monovalent issu d'un cycloalcène comprenant de 3 à 20 atomes de carbone. Les substituants alkyle en $C_1$-$C_{20}$, alkényle en $C_2$-$C_{20}$, alkynyle en $C_2$-$C_{20}$, cycloalkyle en $C_3$-$C_{20}$, cycloalkényle en $C_3$-$C_{20}$, aryle en $C_6$-$C_{18}$ peuvent être substitués ou non par un ou plusieurs substituants -OH, halogène, -$NR^aC(O)R^b$, -$C(O)NR^aR^b$, -CN, -$NO_2$, -$NR^aR^b$, -$OR^a$, -$SR^a$, -$CO_2R^a$, -$OC(O)OR^a$, -$OC(O)R^a$, -$C(O)H$, -$C(O)R^a$ dans lequel $R^a$ et $R^b$ sont indépendamment l'un de l'autre hydrogène, alkyle en $C_1$-$C_{20}$ non substitué, alkényle en $C_2$-$C_{20}$ non substitué, alkynyle en $C_2$-$C_{20}$ non substitué, cycloalkyle en $C_3$-$C_{20}$ non substitué, cycloalkényle en $C_3$-$C_{20}$ non substitué, aryle en $C_6$-$C_{18}$ non substitué. Dans les substituants -$NR^aR^b$, -$NR^aC(O)R^b$, -$C(O)NR^aR^b$, $R^a$ et $R^b$ peuvent former avec l'atome d'azote ou avec le groupement fonctionnel auquel ils sont rattachés un hétérocycle saturé ou insaturé, aromatique ou non, comprenant de 4 à 10 chaînons.

Le terme « hydrohalocarbures » se réfère à des composés de formule $R^aX$ dans laquelle $R^a$ est sélectionné parmi alkyle en $C_1$-$C_{20}$, alkényle en $C_2$-$C_{20}$, alkynyle en $C_2$-$C_{20}$, cycloalkyle en $C_3$-$C_{20}$, cycloalkényle en $C_3$-$C_{20}$, aryle en $C_6$-$C_{18}$ et X représente un atome de chlore, de fluor, de brome ou d'iode. Les substituants alkyle en $C_1$-$C_{20}$, alkényle en $C_2$-$C_{20}$, alkynyle en $C_2$-$C_{20}$, cycloalkyle en $C_3$-$C_{20}$, cycloalkényle en $C_3$-$C_{20}$, aryle en $C_6$-$C_{18}$ peuvent être substitués ou non par un ou plusieurs substituants -OH, halogène, -$NR^aC(O)R^b$, -$C(O)NR^aR^b$ -CN, -$NO_2$, -$NR^aR^b$, -$OR^a$, -$SR^a$, -$CO_2R^a$, -$OC(O)OR^a$, -$OC(O)R^a$, -$C(O)H$, -$C(O)R^a$ dans lequel $R^a$ et $R^b$ sont tels que définis ci-dessus.

Le terme « alcool » se réfère à des hydrocarbures ou hydrohalocarbures tels que définis ci-dessus dans lequel au moins un atome d'hydrogène est remplacé par un groupement hydroxyle -OH.

Le terme « cétone » se réfère à des hydrocarbures comprenant au moins un ou plusieurs groupements fonctionnels carbonyle $R^c$-$C(O)$-$R^d$ dans lequel $R^c$ et $R^d$ sont indépendamment l'un de l'autre un alkyle en $C_1$-$C_{20}$, alkényle en $C_2$-$C_{20}$, alkynyle en $C_2$-$C_{20}$, cycloalkyle en $C_3$-$C_{20}$, cycloalkényle en $C_3$-$C_{20}$, aryle en $C_6$-$C_{18}$ peuvent être substitués ou non par un ou plusieurs substituants -OH, halogène, -$NR^aC(O)R^b$, -$C(O)NR^aR^b$ -CN, -$NO_2$, -$NR^aR^b$, -$OR^a$, -$SR^a$, -$CO_2R^a$, - $OC(O)OR^a$, -$OC(O)R^a$, -$C(O)H$, -$C(O)R^a$ dans lequel $R^a$ et $R^b$ sont tels que définis ci-dessus, $R^c$ et $R^d$ pouvant être liés entre eux pour former avec le groupement carbonyle auquel ils sont rattachés une cétone cyclique comprenant de 4 à 10 chaînons, de préférence de 4 à 7 chaînons. La cétone cyclique peut également comprendre une ou plusieurs doubles liaisons carbone-carbone. La cétone cyclique peut également être substituée ou non par un ou plusieurs substituants tels que définis ci-dessus.

Le terme « amine » se réfère à des hydrocarbures comprenant au moins un ou plusieurs groupements fonctionnels amine -$NR^cR^d$ dans lequel $R^c$ et $R^d$ sont tels que définis ci-dessus, $R^c$ et $R^d$ pouvant être liés entre eux pour former avec l'atome d'azote auquel ils sont rattachés un hétérocycle aromatique ou non comprenant de 4 à 10 chaînons.

Le terme « esters » se réfère à des composés de formule $R^c$-$C(O)$-$O$-$R^d$ dans lequel $R^c$ et $R^d$ sont tels que définis ci-dessus, $R^c$ et $R^d$ pouvant être liés entre eux pour former avec le groupement ester un cycle comprenant de 4 à 20 atomes de carbone.

Le terme « éther » se réfère à des composés de formule $R^c$-$O$-$R^d$ dans lequel $R^c$ et $R^d$ sont tels que définis ci-dessus, $R^c$ et $R^d$ pouvant être liés entre eux pour former avec l'atome d'oxygène auquel ils sont rattachés un hétérocycle comprenant de 4 à 20 atomes de carbone.

Le terme « aldéhyde » se réfère à des composés comprenant au moins un ou plusieurs groupements fonctionnels -$C(O)$-$H$.

Le terme « nitrile » se réfère à des composés comprenant au moins un ou plusieurs groupements fonctionnels -CN.

Le terme « carbonate » se réfère à des composés de formule $R^c$-$O$-$C(O)$-$O$-$R^d$ dans lequel $R^c$ et $R^d$ sont tels que définis ci-dessus.

Le terme « thioalkyle » concerne des composés de formule $R^cSR^d$ dans laquelle $R^c$ et $R^d$ sont tels que définis ci-dessus.

Le terme « amide » concerne des composés de formule $R^cC(O)NR^eR^d$ dans laquelle $R^c$ et $R^d$ sont tels que définis ci-dessus, $R^e$ étant choisi parmi les mêmes substituants que ceux définissant $R^c$, $R^c$ et $R^d$ pouvant être liés entre eux pour former avec le groupement amide - $C(O)N$- auquel ils sont rattachés une amide cyclique comprenant de 4 à 10 chaînons, de préférence de 4 à 7 chaînons. L'amide cyclique peut également comprendre une ou plusieurs doubles liaisons carbone-carbone. L'amide cyclique peut également être substituée ou non par un ou plusieurs substituants tels que définis ci-dessus.

Le terme « hétérocycle » désigne un cycle carboné comprenant de 4 à 10 chaînons dont au moins un des chaînons est un hétéroatome sélectionné parmi le groupe consistant en O, S, P et N. L'hétérocycle peut comprendre un ou plusieurs double liaison carbone-carbone ou une ou plusieurs double liaison carbone-hétéroatome ou une ou plusieurs double liaison hétéroatome-hétéroatome. De préférence, l'hétérocycle peut comprendre 1, 2, 3, 4 ou 5 hétéroatomes tel que défini ci-dessus. En particulier, l'hétérocycle peut comprendre 1, 2 ou 3 hétéroatomes sélectionné parmi l'oxygène, l'azote ou le soufre. De préférence, l'hétérocycle peut être un cycle carboné comprenant de 4 à 6 chaînons dont 1, 2 ou 3 chaînons sont des hétéroatomes sélectionnés parmi O ou N. L'hétérocycle peut être ou non substitué par un ou plusieurs substituants choisi parmi -OH, halogène, -$NR^aC(O)R^b$, -$C(O)NR^aR^b$ -CN, -$NO_2$, -$NR^aR^b$,

-OR$^a$, -SR$^a$, - CO$_2$R$^a$, -OC(O)OR$^a$, -OC(O)R$^a$, -C(O)H, -C(O)R$^a$ dans lequel R$^a$ et R$^b$ sont tels que définis ci-dessus.

Le terme « agent d'extraction organique » se réfère à un composé comportant au moins un atome de carbone.

Selon un premier aspect, l'invention se rapporte à un procédé de purification du 2,3,3,3-tétrafluoro-1-propene (1234yf). Le procédé de purification est effectué à partir d'une première composition comprenant du 2,3,3,3-tétrafluoro-1-propène, 1,1,1,2,2-pentafluoropropane (245cb) et trans-1,3,3,3-tétrafluoro-1-propène (1234ze-E).

De préférence, ledit procédé comprend les étapes de :

a) mise en contact de ladite première composition avec au moins un agent d'extraction organique pour former une seconde composition ;

b) distillation extractive de ladite seconde composition pour former :

i) une troisième composition comprenant ledit agent d'extraction organique et trans-1,3,3,3-tetrafluoro-1-propene (1234ze-E) et

ii) un courant comprenant le 2,3,3,3-tétrafluoro-1-propène et 1,1,1,2,2-pentafluoropropane (245cb),

c) récupération et séparation de ladite troisième composition pour former un courant comprenant ledit agent d'extraction organique et un courant comprenant le trans-1,3,3,3-tetrafluoro-1-propene (1234ze-E).

De préférence, le courant comprenant ledit agent d'extraction organique est recyclé à l'étape a). De préférence, le courant comprenant le trans-1,3,3,3-tetrafluoro-1-propène (1234ze-E) est purifié ou détruit par incinération.

De préférence, ledit procédé comprend également une étape de récupération dudit courant comprenant le 2,3,3,3-tetrafluoro-1-propène (1234yf) et le 1,1,1,2,2-pentafluoropropane (245cb) obtenu à l'étape b), et distillation de celui-ci pour former un courant A comprenant 2,3,3,3-tetrafluoro-1-propène et un courant B comprenant 1,1,1,2,2-pentafluoropropane (245cb).

Ladite première composition peut comprendre du 2,3,3,3-tétrafluoro-1-propène, 1,1,1,2,2-pentafluoropropane (245cb), trans-1,3,3,3-tetrafluoro-1-propene (1234ze-E) et au moins un des composés sélectionné parmi le groupe consistant en chlorométhane (40), 1,1-difluoroéthane (152a), 1,1,1,2-tetrafluoroethane (134a), chloropentafluoroéthane (115), trans-1,2,3,3,3-pentafluoropropene (1225ye-E), cis-1,2,3,3,3-pentafluoropropene (1225ye-Z), 3,3,3-trifluoropropene (1243zf). De préférence, la première composition peut comprendre au moins deux, au moins trois, au moins quatre, au moins cinq, au moins six ou l'ensemble des composés sélectionné parmi le groupe consistant en chlorométhane (40), 1,1-difluoroéthane (152a), 1,1,1,2-tetrafluoroethane (134a), chloropentafluoroéthane (115), trans-1,2,3,3,3-pentafluoropropene (1225ye-E), cis-1,2,3,3,3-pentafluoropropene (1225ye-Z), 3,3,3-trifluoropropene (1243zf).

Lorsque ladite première composition contient au moins un des composés sélectionné parmi le groupe consistant en chlorométhane (40), 1,1-difluoroéthane (152a), 1,1,1,2-tetrafluoroethane (134a), chloropentafluoroéthane (115), trans-1,2,3,3,3-pentafluoropropene (1225ye-E), cis-1,2,3,3,3-pentafluoropropene (1225ye-Z), 3,3,3-trifluoropropene (1243zf), celui-ci ou ceux-ci sont contenus dans lesdites seconde et troisième compositions.

L'étape c) du présent procédé peut ainsi consister en la récupération de ladite troisième composition et la séparation entre d'une part ledit agent d'extraction organique et d'autre part le trans-1,3,3,3-tetrafluoro-1-propene (1234ze-E) et ledit au moins un des composés sélectionné parmi le groupe consistant en chlorométhane (40), 1,1-difluoroéthane (152a), 1,1,1,2-tetrafluoroethane (134a), chloropentafluoroéthane (115), trans-1,2,3,3,3-pentafluoropropene (1225ye-E), cis-1,2,3,3,3-pentafluoropropene (1225ye-Z), 3,3,3-trifluoropropene (1243zf).

Ladite première composition peut comprendre entre 50 et 99 % en poids de 2,3,3,3-tétrafluoro-1-propène sur base du poids total de la première composition, avantageusement entre 50 et 95% en poids, de préférence entre 55 et 90% et en particulier entre 60 et 80% en poids de 2,3,3,3-tétrafluoro-1-propène sur base du poids total de la première composition.

Lorsqu'elle en contient, ladite première composition peut comprendre entre 0.1 et 50 % en poids de 1,1,1,2,2-pentafluoropropane (245cb) sur base du poids total de la première composition, avantageusement entre 0,1 et 49,9 % en poids, de préférence entre 1 et 45% en poids, plus préférentiellement entre 2 et 40% et en particulier entre 5 et 40% en poids de 1,1,1,2,2-pentafluoropropane (245cb) sur base du poids total de la première composition.

Lorsqu'elle en contient, ladite première composition peut comprendre entre 0.1 et 50 % en poids de trans-1,3,3,3-tetrafluoro-1-propene (1234ze-E) sur base du poids total de la première composition, avantageusement entre 0,1 et 49,9 % en poids, de préférence entre 0.5 et 40% en poids, plus préférentiellement entre 1 et 30% et en particulier entre 5 et 25% en poids de trans-1,3,3,3-tetrafluoro-1-propene (1234ze-E) sur base du poids total de la première composition.

Lorsqu'elle en contient, ladite première composition peut comprendre moins de 1% poids de chlorométhane (40) sur base du poids total de la première composition, avantageusement entre 1 et 5000 ppm poids, de préférence entre 5 et 2000 ppm et en particulier entre 10 et 1000 ppm en poids de chlorométhane (40) sur base du poids total de la première composition.

Lorsqu'elle en contient, ladite première composition peut comprendre moins de 2000 ppm poids de 1,1-difluoroéthane (152a) sur base du poids total de la première composition, avantageusement entre 1 et 1000 ppm poids, de préférence entre 5 et 500 ppm et en particulier entre 10 et 250 ppm en poids de 1,1-difluoroéthane (152a) sur base du poids total de la première composition.

Lorsqu'elle en contient, ladite première composition peut comprendre moins de 2000 ppm poids de chloropentafluoroéthane (115) sur base du poids total de la première composition, avantageusement entre 1 et 1000 ppm poids, de préférence entre 5 et 500 ppm et en particulier entre 10 et 250 ppm en poids de chloropentafluoroéthane (115) sur base du poids total de la première composition.

Lorsqu'elle en contient, ladite première composition peut comprendre moins de 2000 ppm poids de 1,1,1,2-tetrafluoroethane (134a) sur base du poids total de la première composition, avantageusement entre 1 et 1000 ppm poids, de préférence entre 5 et 500 ppm et en particulier entre 10 et 250 ppm poids de 1,1,1,2-tetrafluoroethane (134a) sur base du poids total de la première composition.

Lorsqu'elle en contient, ladite première composition peut comprendre moins de 2000 ppm poids de trans-1,2,3,3,3-pentafluoropropene (1225ye-E) sur base du poids total de la première composition, avantageusement entre 1 et 1000 ppm poids, de préférence entre 5 et 500 ppm et en particulier entre 10 et 250 ppm poids de trans-1,2,3,3,3-pentafluoropropene (1225ye-E) sur base du poids total de la première composition.

Lorsqu'elle en contient, ladite première composition peut comprendre moins de 2000 ppm poids de cis-1,2,3,3,3-pentafluoropropene (1225ye-Z) sur base du poids total de la première composition, avantageusement entre 1 et 1000 ppm poids, de préférence entre 5 et 500 ppm et en particulier entre 10 et 250 ppm poids de cis-1,2,3,3,3-pentafluoropropene (1225ye-Z) sur base du poids total de la première composition.

Lorsqu'elle en contient, ladite première composition peut comprendre moins de 1 % en poids de 3,3,3-trifluoropropene (1243zf) sur base du poids total de la première composition, avantageusement entre 1 et 5000 ppm poids, de préférence entre 5 et 2000 ppm et en particulier entre 10 et 1500 ppm poids de 3,3,3-trifluoropropene (1243zf) sur base du poids total de la première composition.

Selon un mode de réalisation particulier, ledit agent d'extraction organique peut être un solvant choisi parmi le groupe consistant en hydrohalocarbure, alcool, cétone, amine, ester, éther, aldéhyde, nitrile, carbonate, thioalkyle, amide, hétérocycle; ou l'agent d'extraction organique est triethylfluorosilane. De préférence, l'agent d'extraction organique est choisi parmi le groupe consistant en amine, éther, ester, aldéhyde, cétone, alcool et hétérocycle.

De préférence, les hydrohalocarbures sont sélectionnés parmi le groupe consistant en bromofluoromethane, 1-bromo-1,2-difluoroethylene, 1,1,1-trifluoro-2-bromoethane, 2-chloropropane, bromoethane, iodomethane, 2-chloro-2-methylpropane, 2-bromopropane, chlorobromomethane, 3-bromopropene, 1-bromopropane, iodoethane, 2-bromo-2-methylpropane, 1-chloro-3-fluoropropane, 2-chloro-2-methylbutane, 1,2-dichloroethane, 2-iodopropane, dichlorobromomethane, 2-bromobutane, 1,2-dichloropropane, trichloroacetaldehyde, 1-chloro-4-fluorobutane, 1-bromo-3-fluoropropane, 1-bromobutane, 1-iodopropane, cis-1,3-dichloropropene, bromotrichloromethane, 1-bromo-2-chloroethane, 2-bromo-2-methylbutane, trans-1,3-dichloropropene, 1,1,2-trichloroethane, 2-bromopentane, 2,3-dichlorobutane, 1-bromo-3-methylbutane, 1,3-dichloro-trans-2-butene, 1,3-dichloropropane, 1,2-dibromo-1-fluoroethane, 1,2,2-trichloropropane, 2,3-dichloro-2-methylbutane, 1-bromopentane, 1,2-dichloro-2-butene, 1-iodobutane, 1,2-dibromoethane, 1,1,2-trichloropropane, 1,3-dichlorobutane, 1,2-dibromopropane, 1,2,3-trichloropropene, 1-chloro-3-bromopropane, tribromomethane.

De préférence, les alcools sont sélectionnés parmi le groupe consistant en méthanol, 2,2,2-trifluoroethanol, 1,1,1-trifluoro-2-propanol, ethanol, 2-propanol, tert-butanol, 2,2-difluoroethanol, propanol, 2-allyloxyethanol, 2-butanol, 2-methyl-2-butanol, isobutanol, 2,2,3,3-tetraflouro-1-propanol, 2,2-dimethyl-1-propanol, 3-pentanol, 1-butanol, 1-methoxy2-propanol, 1-(dimethylamino)-2-propanol, 3-methyl-3-pentanol, 1-chloro-2-methyl-2-propanol, 4,4,4-trifluorobutanol, 3-fluoropropanol, 2-chloroethanol, 2-methoxy1-propanol, 1-ethoxy-2-propanol, 4-methyl-2-pentanol, 1,2-octanediol, 2-chloro-1-propanol, 2-(dimethylamino)-ethanol, 3-hexanol, 2-hexanol, 2-ethoxy-1-propanol, 1-pentanol, 2,3-dimethylbutanol, 2-ethyl-1-butanol, 2-methyl-1-pentanol, 2-propoxyethanol.

De préférence, les cétones sont sélectionnées parmi le groupe consistant en 1,1,1-trifluoro-2-propanone, propanone, butanone, 3-pentanone, 2-pentanone, 3,3-dimethyl-2-butanone, 4-methyl-2-pentanone, 2-hexanone, 5-hexen-2-one, 4-methyl-2-hexanone.

De préférence, les amines sont sélectionnées parmi le groupe consistant en Ethylamine, isopropylamine, ethylmethylamine, 2-amino-2-methylpropane, n-propylamine, isopropylmethylamine, diethylamine, 2-butanamine, n-methylpropylamine, 1-butylamine, diisopropylamine, 3-methyl-2-butanamine, 3-pentylamine, n-methylbutylamine, 1-methoxy-2-propanamine, 2-methoxyethanamine, 2-methoxy-1propanamine, n-pentylamine, n-methylhydroxylamine, dipropylamine, 2-ethoxyethanamine, n-methyl-1,2-ethanediamine, pyridine, 1,2-diaminoethane, 1,2-propanediamine, 2-ethylbutylamine, n-ethylethylenediamine, 1,1-diethoxy-n,n-dimethylmethanamine, 2-methylpyridine, 4-methyl-2-hexanamine, hexylamine, cyclohexylamine, n-ethyl-2-dimethylaminoethylamine, 1,3-propanediamine, 2-heptanamine, n,n-diethylethylenediamine, 2,6-dimethylpyridine, 4-methylpyridine, n,n'-diethyl-1,2-ethanediamine.

De préférence, les esters sont sélectionnés parmi le groupe consistant Methylformate, methylacetate, isopropylformate, ethylacetate, n-propylformate, isopropylacetate, tert-butylacetate, ethylpropionate, sec-butylacetate, diethylcarbonate, n-butylacetate, bromoaceticacidmethylester, methylhexanoate.

De préférence, les éthers sont sélectionnés parmi le groupe consistant en 2,2,2-trifluoroethylmethylether, 2-methoxy-1-propene, diethylether, ethoxy-ethene, dimethoxymethane, methylcyclopropylether, 2-ethoxy-propane, methyl-t-butylether, chloromethoxymethane, diisopropylether, 2-ethoxy-2-methyl-propane, 2-ethoxy-butane, 1-methoxy-2-methyl-butane, 2,2-dimethoxypropane, 1-ethoxy-2-methylpropane, 1,2-dimethoxyethane, diethoxymethane, di-n-propylether, 1-ethoxybutane, 1-methoxy-pentane, 1,2-dimethoxypropane,, isopropyl-isobutyl-ether, 1,1-diethoxyethane, trimethoxymethane, 2,2-diethoxypropane, isobutyl-tert-butylether, sec-butyl-tert-butylether, 1,1-diethoxypropane, 2-methoxyethanol, 2-chloro-1,1-dimethoxyethane, methoxycyclohexane, ethoxyethanol, di-n-butylether, 1-ethoxy-hexane, 1,1,1-triethoxyethane, 1-methoxy-2-acetoxypropane.

De préférence, les aldéhydes sont sélectionnés parmi le groupe consistant en acétaldehyde, ethanedial, isobutanal, methylglyoxal, 2-methylbutanal, 2,6-dimethyl-5-heptenal, hexanal.

De préférence, les nitriles sont sélectionnés parmi le groupe consistant en acétonitrile, propionitrile, butyronitrile, valeronitrile, (methyleneamino)acetonitrile.

De préférence, le carbonate est le diéthylcaarbonate.

De préférence, l'amide est l'éthanethioamide.

De préférence, les thioalkyles sont sélectionnés parmi le groupe consistant en éthanethiol, dimethylsulfide, 2-propanethiol, 4-methoxy-2-methyl-2-butanethiol, tert-butylthiol, 1-propanethiol, 2-butanethiol, 2-methyl-1-propanethiol, diethylsulfide, butanethiol, 3-mercapto-1,2-propanediol, tetrahydrothiophene, 1-pentanethiol.

De préférence, les hétérocycles sont choisis parmi le groupe consistant en tétrahydrofurane, dioxane, 1,3-dioxane, 1,3,5-trioxane, n-methylmorpholine, 2-methylpyrazine, n-ethyl-morpholine, 1-methylpiperazine, 1,2-epoxypropane, piperidine, 3-furfural, 2,6-dimethylmorpholine.

Ledit agent d'extraction organique peut être éthylamine, bromofluoromethane, 1-bromo-1,2-difluoroethylene, acetaldehyde, 1,1,1-trifluoro-2-propanone, 1,1,1-trifluoro-2-bromoethane, 2,2,2-trifluoroethylmethylether, isopropylamine, methylformate, 2-methoxy-1-propene, diethylether, 1,2-epoxypropane, ethanethiol, ethoxy-ethene, ethylmethylamine, dimethylsulfide, 2-chloropropane, bromoethane, dimethoxymethane, iodomethane, 2-amino-2-methylpropane, methylcyclopropylether, n-propylamine, isopropylmethylamine, ethanedial, 2-chloro-2-methylpropane, 2-propanethiol, 2-ethoxy-propane, methyl-t-butylether, diethylamine, propanone, methylacetate, 4-methoxy-2-methyl-2-butanethiol, 2-bromopropane, chloromethoxymethane, 2-butanamine, n-methylpropylamine, tert-butylthiol, isobutanal, methanol, tetrahydrofurane, 1-propanethiol, chlorobromomethane, isopropylformate, diisopropylether, 3-bromopropene, 1-bromopropane, methylglyoxal, iodoethane, 2-ethoxy-2-methyl-propane, 2-bromo-2-methylpropane, 2,2,2-trifluoroethanol, 1-chloro-3-fluoropropane, 1,1,1-trifluoro-2-propanol, 1-butylamine, ethylacetate, ethanol, butanone, n-propylformate, 2-ethoxy-butane, 2-propanol, acetonitrile, tert-butanol, 1-methoxy-2-methyl-butane, 2,2-dimethoxypropane, 2-chloro-2-methylbutane, 1,2-dichloroethane, 1-ethoxy-2-methylpropane, diisopropylamine, 2-butanethiol, 1,2-dimethoxyethane, 3-methyl-2-butanamine, diethoxymethane, 2-methyl-1-propanethiol, isopropylacetate, 2-iodopropane, di-n-propylether, 3-pentylamine, n-methylbutylamine, 2-bromobutane, diethylsulfide, 1-ethoxybutane, 1-methoxy-2-propanamine, 2-methylbutanal, 2-methoxyethanamine, 2,2-difluoroethanol, 1,2-dichloropropane, propanol, tert-butylacetate, propionitrile, trichloroacetaldehyde, 2-allyloxyethanol, butanethiol, 1-methoxy-pentane, ethylpropionate, 2-butanol, 1,2-dimethoxypropane, isopropyl-isobutyl-ether, 1-chloro-4-fluorobutane, 1-bromo-3-fluoropropane, dioxane, 1-bromobutane, 3-pentanone, 1,1-diethoxyethane, 2-pentanone, 2-methyl-2-butanol, 1-iodopropane, 2-methoxy-1propanamine, trimethoxymethane, cis-1,3-dichloropropene, n-pentylamine, 3,3-dimethyl-2-butanone, 1,3-dioxane, piperidine, 1-bromo-2-chloroethane, isobutanol, 2-bromo-2-methylbutane, dipropylamine, 2,2,3,3-tetraflouro-1-propanol, 2-ethoxyethanamine, triethylfluorosilane, sec-butylacetate, trans-1,3-dichloropropene, 2,2-dimethyl-1-propanol, n-methyl-1,2-ethanediamine, 2,2-diethoxypropane, 1,3,5-trioxane, pyridine, n-methylmorpholine, 3-pentanol, 4-methyl-2-pentanone, 1,2-diaminoethane, isobutyl-tert-butylether, 2-bromopentane, butyronitrile, 1-butanol, 2,3-dichlorobutane, sec-butyl-tert-butylether, 1-methoxy2-propanol, 1,2-propanediamine, 2,6-dimethyl-5-heptenal, 1-bromo-3-methylbutane, 1,3-dichloro-trans-2-butene, 1,3-dichloropropane, 1-(dimethylamino)-2-propanol, tetrahydrothiophene, 3-methyl-3-pentanol, 1,2-dibromo-1-fluoroethane, 1,1-diethoxypropane, 1,2,2-trichloropropane, 1-chloro-2-methyl-2-propanol, 2-methoxyethanol, 4,4,4-trifluorobutanol, 2-ethylbutylamine, diethylcarbonate, n-butylacetate, 1-pentanethiol, 2-chloro-1,1-dimethoxyethane, 2-hexanone, n-ethylethylenediamine, 3-fluoropropanol, 5-hexen-2-one, 2,3-dichloro-2-methylbutane, 1,1-diethoxy-n,n-dimethylmethanamine, 2-methylpyridine, 1-bromopentane, 2-methoxy1-propanol, 1,2-dichloro-2-butene, 1-iodobutane, hexanal, 1-ethoxy-2-propanol, 1,2-dibromoethane, 4-methyl-2-pentanol, bromoaceticacidmethylester, 1,1,2-trichloropropane, 1,2-octanediol, 4-methyl-2-hexanamine, hexylamine, 2-chloro-1-propanol, methoxycyclohexane, 2-(dimethylamino)-ethanol, 1,3-dichlorobutane, cyclohexylamine, n-ethyl-2-dimethylaminoethylamine, ethoxyethanol, 3-hexanol, 2-hexanol, 2-methylpyrazine, 2-ethoxy-1-propanol, 1-pentanol, n-ethyl-morpholine, 1-methylpiperazine, 1,3-propanediamine, di-n-butylether, valeronitrile, (methyleneamino)acetonitrile, 1,2-dibromopropane, 1,2,3-trichloropropane, 2-heptanamine, 2,3-dimethylbutanol, 1-ethoxy-hexane, 1-chloro-3-bromopropane, n,n-diethylethylenediamine, 3-furfural, 2,6-dimethylpyridine, 4-methyl-2-hexanone, 1,1,1-triethoxyethane, 1-methoxy-2-acetoxypropane, 4-methylpyridine, n,n'-diethyl-1,2-ethanediamine, 2,6-dimethylmorpholine, 2-ethyl-1-butanol, 2-methyl-1-pentanol, methylhexanoate, 2-propoxyethanol, 1-propoxy-2-propanol, dimethylethanolamine.

Avantageusement, ledit agent d'extraction organique peut être éthylamine, bromofluoromethane, acetaldehyde, isopropylamine, methylformate, 2-methoxy-1-propene, diethylether, 1,2-epoxypropane, ethanethiol, ethoxy-ethene, ethylmethylamine, dimethylsulfide, bromoethane, dimethoxymethane, 2-amino-2-methylpropane, methylcyclopropylether, n-propylamine, isopropylmethylamine, 2-propanethiol, 2-ethoxy-propane, methyl-t-butylether, diethylamine, propanone, methylacetate, 4-methoxy-2-methyl-2-butanethiol, 2-bromopropane, 2-butanamine, n-methylpropylamine, tert-butylthiol, isobutanal, tetrahydrofurane, 1-propanethiol, isopropylformate, diisopropylether, 1-bromopropane, methylglyoxal, 2-ethoxy-2-methyl-propane, 2-bromo-2-methylpropane, 1-butylamine, ethylacetate, butanone, n-propylformate, 2-ethoxy-butane, 2-propanol, tert-butanol, 1-methoxy-2-methyl-butane, 2,2-dimethoxypropane, 1-ethoxy-2-methylpropane, diisopropylamine, 2-butanethiol, 1,2-dimethoxyethane, 3-methyl-2-butanamine, diethoxymethane, 2-methyl-1-propanethiol, isopropylacetate, 2-iodopropane, di-n-propylether, 3-pentylamine, n-methylbutylamine, 2-bromobutane, diethylsulfide, 1-ethoxybutane, 1-methoxy-2-propanamine, 2-methylbutanal, 2-methoxyethanamine, propanol, tert-butylacetate, propionitrile, 2-allyloxyethanol, butanethiol, 1-methoxy-pentane, ethylpropionate, 2-butanol, 1,2-dimethoxypropane, isopropyl-isobutyl-ether, 1-bromo-3-fluoropropane, dioxane, 1-bromobutane, 3-pentanone, 1,1-diethoxyethane, 2-pentanone, 2-methyl-2-butanol, 2-methoxy-1propanamine, trimethoxymethane, n-pentylamine, 3,3-dimethyl-2-butanone, 1,3-dioxane, piperidine, isobutanol, 2-bromo-2-methylbutane, dipropylamine, 2-ethoxyethanamine, sec-butylacetate, 2,2-dimethyl-1-propanol, n-methyl-1,2-ethanediamine, 2,2-diethoxypropane, 1,3,5-trioxane, pyridine, n-methylmorpholine, 3-pentanol, 4-methyl-2-pentanone, 1,2-diaminoethane, isobutyl-tert-butylether, 2-bromopentane, butyronitrile, 1-butanol, sec-butyl-tert-butylether, 1-methoxy2-propanol, 1,2-propanediamine, 2,6-dimethyl-5-heptenal, 1-bromo-3-methylbutane, 1-(dimethylamino)-2-propanol, tetrahydrothiophene, 3-methyl-3-pentanol, 1,1-diethoxypropane, 2-methoxyethanol, 2-ethylbutylamine, diethylcarbonate, n-butylacetate, 1-pentanethiol, 2-chloro-1,1-dimethoxyethane, 2-hexanone, n-ethylethylenediamine, 5-hexen-2-one, 1,1-diethoxy-n,n-dimethylmethanamine, 2-methylpyridine, 1-bromopentane, 2-methoxy1-propanol, hexanal, 1-ethoxy-2-propanol, 4-methyl-2-pentanol, 1,2-octanediol, 4-methyl-2-hexanamine, hexylamine, methoxycyclohexane, 2-(dimethylamino)-ethanol, cyclohexylamine, n-ethyl-2-dimethylaminoethylamine, ethoxyethanol, 3-hexanol, 2-hexanol, 2-methylpyrazine, 2-ethoxy-1-propanol, 1-pentanol, n-ethyl-morpholine, 1-methylpiperazine, 1,3-propanediamine, di-n-butylether, valeronitrile, (methyleneamino)acetonitrile, 2-heptanamine, 2,3-dimethylbutanol, 1-ethoxy-hexane, n,n-diethylethylenediamine, 3-furfural, 2,6-dimethylpyridine, 4-methyl-2-hexanone, 1,1,1-triethoxyethane, 1-methoxy-2-acetoxypropane, 4-methylpyridine, n,n'-diethyl-1,2-ethanediamine, 2,6-dimethylmorpholine, 2-ethyl-1-butanol, 2-methyl-1-pentanol, methylhexanoate, 2-propoxyethanol, 1-propoxy-2-propanol, dimethylethanolamine

De préférence, ledit agent d'extraction organique peut être éthylamine, acetaldehyde, isopropylamine, methylformate, diethylether, 1,2-epoxypropane, ethylmethylamine, dimethoxymethane, 2-amino-2-methylpropane, methylcyclopropylether, n-propylamine, isopropylmethylamine, 2-ethoxy-propane, methyl-t-butylether, diethylamine, propanone, methylacetate, 4-methoxy-2-methyl-2-butanethiol, 2-butanamine, n-methylpropylamine, isobutanal, tetrahydrofurane, isopropylformate, diisopropylether, 2-ethoxy-2-methyl-propane, 1-butylamine, ethylacetate, butanone, n-propylformate, 2-ethoxy-butane, 1-methoxy-2-methyl-butane, 2,2-dimethoxypropane, 1-ethoxy-2-methylpropane, diisopropylamine, 1,2-dimethoxyethane, 3-methyl-2-butanamine, diethoxymethane, isopropylacetate, di-n-propylether, 3-pentylamine, n-methylbutylamine, diethylsulfide, 1-ethoxybutane, 1-methoxy-2-propanamine, 2-methylbutanal, 2-methoxyethanamine, tert-butylacetate, propionitrile, 2-allyloxyethanol, 1-methoxy-pentane, ethylpropionate, 1,2-dimethoxypropane, isopropyl-isobutyl-ether, dioxane, 3-pentanone, 1,1-diethoxyethane, 2-pentanone, 2-methoxy-1propanamine, trimethoxymethane, n-pentylamine, 3,3-dimethyl-2-butanone, 1,3-dioxane, piperidine, dipropylamine, 2-ethoxyethanamine, sec-butylacetate, n-methyl-1,2-ethanediamine, 2,2-diethoxypropane, pyridine, n-methylmorpholine, 4-methyl-2-pentanone, 1,2-diaminoethane, isobutyl-tert-butylether, butyronitrile, sec-butyl-tert-butylether, 1-methoxy2-propanol, 1,2-propanediamine, 2,6-dimethyl-5-heptenal, 1-(dimethylamino)-2-propanol, 3-methyl-3-pentanol, 1,1-diethoxypropane, 2-ethylbutylamine, diethylcarbonate, n-butylacetate, 2-hexanone, n-ethylethylenediamine, 5-hexen-2-one, 1,1-diethoxy-n,n-dimethylmethanamine, 2-methylpyridine, 2-methoxy1-propanol, hexanal, 1-ethoxy-2-propanol, 4-methyl-2-hexanamine, hexylamine, methoxycyclohexane, 2-(dimethylamino)-ethanol, cyclohexylamine, n-ethyl-2-dimethylaminoethylamine, ethoxyethanol, 2-methylpyrazine, 2-ethoxy-1-propanol, n-ethyl-morpholine, 1-methylpiperazine, 1,3-propanediamine, di-n-butylether, valeronitrile, 2-heptanamine, 1-ethoxy-hexane, n,n-diethylethylenediamine, 2,6-dimethylpyridine, 4-methyl-2-hexanone, 1,1,1-triethoxyethane, 1-methoxy-2-acetoxypropane, 4-methylpyridine, n,n'-diethyl-1,2-ethanediamine, 2,6-dimethylmorpholine, methylhexanoate, 2-propoxyethanol, 1-propoxy-2-propanol.

En particulier, ledit agent d'extraction organique peut être éthylamine, isopropylamine, diethylether, ethylmethylamine, 2-amino-2-methylpropane, n-propylamine, isopropylmethylamine, 2-ethoxy-propane, methyl-t-butylether, diethylamine, propanone, methylacetate, 2-butanamine, n-methylpropylamine, isobutanal, tetrahydrofurane, 1-butylamine, ethylacetate, butanone, n-propylformate, 2,2-dimethoxypropane, 1-ethoxy-2-methylpropane, 1,2-dimethoxyethane, 3-methyl-2-butanamine, diethoxymethane, isopropylacetate, 3-pentylamine, n-methylbutylamine, 1-ethoxybutane, 1-methoxy-2-propanamine, 2-methylbutanal, 2-methoxyethanamine, tert-butylacetate, 1-methoxy-pentane, ethylpropionate, 1,2-dimethoxypropane, dioxane, 3-pentanone, 1,1-diethoxyethane, 2-pentanone, 2-methoxy-1propanamine, trimethoxymethane, n-pentylamine, 3,3-dimethyl-2-butanone, 1,3-dioxane, piperidine, 2-ethoxyethanamine, sec-butylacetate, n-methyl-1,2-ethanediamine, 2,2-diethoxypropane, 4-methyl-2-pentanone, 1,2-diaminoethane, butyronitrile, 1-methoxy2-propanol, 1,2-propanediamine, 2,6-dimethyl-5-heptenal, 1-(dimethylamino)-2-propanol, 1,1-diethoxypropane, 2-ethylbutylamine, diethylcarbonate, n-butylacetate, 2-hexanone, n-ethylethylenediamine, 5-hexen-2-one, 2-methylpyridine, 2-methoxy1-propanol, hexanal, 1-ethoxy-2-propanol, 4-methyl-2, hexanamine, hexylamine, methoxycyclohexane, 2-(dimethylamino)-ethanol, cyclohexylamine, n-ethyl-2-dimethylaminoethylamine, 2-methylpyrazine, 2-ethoxy-1-propanol, 1-methylpiperazine, 1,3-propanediamine, di-n-butylether, valeronitrile, 2-heptanamine, 1-ethoxy-hexane, n,n-diethylethylenediamine, 2,6-dimethylpyridine, 4-methyl-2-hexanone, 1,1,1-triethoxyethane, 1-methoxy-2-acetoxypropane, 4-methylpyridine, n,n'-diethyl-1,2-ethanediamine, 2,6-dimethylmorpholine, methylhexanoate, 2-propoxyethanol, 1-propoxy-2-propanol.

L'agent d'extraction organique à utiliser peut être sélectionné en fonction des composés présents dans ladite première composition. Ainsi, l'agent d'extraction organique peut être sélectionné en fonction du facteur de séparation et de la capacité d'absorption établi pour une composition particulière. Outre ces deux critères, le choix de l'agent d'extraction organique peut se baser optionnellement sur d'autres critères commerciaux ou environnementaux, tels que par exemple le coût de l'agent d'extraction organique, sa disponibilité sur le marché, ses propriétés toxiques ou inflammable. De plus, selon un mode de réalisation particulier, afin d'optimiser le fonctionnement des colonnes de distillation utilisées à l'étape b) et c) du présent procédé de purification du 2,3,3,3-tetrafluoro-1-propene, le point d'ébullition de l'agent d'extraction organique peut être de 0°C à 200°C, avantageusement de 10°C à 190°C, de préférence de 10°C à 180°C, en particulier de 10°C à 170°C, plus particulièrement de 10°C à 160°C, et de manière privilégiée de 10°C à 150°C.

Selon un mode de réalisation préféré, ledit agent d'extraction organique a un facteur de séparation $S_{1,2}$ supérieur ou égal à 1,1, ledit facteur de séparation étant calculé par la formule $S_{1,2} = (\gamma_{1,S} * P1)/(\gamma_{2,S} * P2)$ dans laquelle

$\gamma_{1,S}$ représente le coefficient d'activité du 2,3,3,3-tétrafluoro-1-propène (1234yf) dans ledit agent d'extraction organique à dilution infinie,
P1 représente la pression de vapeur saturante du 2,3,3,3-tétrafluoro-1-propène (1234yf),
$\gamma_{2,S}$ représente le coefficient d'activité du trans-1,3,3,3-tetrafluoro-1-propene (1234ze-E) dans ledit agent d'extraction organique à dilution infinie,
P2 représente la pression de vapeur saturante du trans-1,3,3,3-tetrafluoro-1-propene (1234ze-E).

Selon un mode de réalisation préféré, ledit agent d'extraction organique a un facteur de séparation $S_{1,2}$ supérieur ou égal à 1,1, ledit facteur de séparation étant calculé par la formule $S_{1,2} = (\gamma_{1,S} * P1)/(\gamma_{2,S} * P2)$ dans laquelle

$\gamma_{1,S}$ représente le coefficient d'activité du 1,1,1,2,2-pentafluoropropane (245cb) dans ledit agent d'extraction organique à dilution infinie,
P1 représente la pression de vapeur saturante du 1,1,1,2,2-pentafluoropropane (245cb), $\gamma_{2,S}$ représente le coefficient d'activité du trans-1,3,3,3-tetrafluoro-1-propene (1234ze-E) dans ledit agent d'extraction organique à dilution infinie,
P2 représente la pression de vapeur saturante du trans-1,3,3,3-tetrafluoro-1-propene (1234ze-E).

Selon un mode de réalisation préféré, ledit agent d'extraction organique a :

- un facteur de séparation $S_{1,2}$ supérieur ou égal à 1,1, ledit facteur de séparation étant calculé par la formule $S_{1,2} = (\gamma_{1,3} * P1)/(\gamma_{2,3} * P2)$ dans laquelle

 $\gamma_{1,S}$ représente le coefficient d'activité du 2,3,3,3-tétrafluoro-1-propène (1234yf) dans ledit agent d'extraction organique à dilution infinie,
 P1 représente la pression de vapeur saturante du 2,3,3,3-tétrafluoro-1-propène (1234yf),
 $\gamma_{2,S}$ représente le coefficient d'activité du trans-1,3,3,3-tetrafluoro-1-propene (1234ze-E) dans ledit agent d'extraction organique à dilution infinie,
 P2 représente la pression de vapeur saturante du trans-1,3,3,3-tetrafluoro-1-propene (1234ze-E) ;
 et

- un facteur de séparation $S_{1,2}$ supérieur ou égal à 1,1, ledit facteur de séparation étant calculé par la formule $S_{1,2} = (\gamma_{1,S} * P1)/(\gamma_{2,S} * P2)$ dans laquelle

 $\gamma_{1,S}$ représente le coefficient d'activité du 1,1,1,2,2-pentafluoropropane (245cb) dans ledit agent d'extraction organique à dilution infinie,
 P1 représente la pression de vapeur saturante du 1,1,1,2,2-pentafluoropropane (245cb), $\gamma_{2,S}$ représente le coefficient d'activité du trans-1,3,3,3-tetrafluoro-1-propene (1234ze-E) dans ledit agent d'extraction organique à dilution infinie,

P2 représente la pression de vapeur saturante du trans-1,3,3,3-tetrafluoro-1-propene (1234ze-E).

Avantageusement, dans les deux cas, le facteur de séparation $S_{1,2}$ peut être supérieur ou égal à 1,2, de préférence supérieur ou égal à 1,4, plus préférentiellement supérieur ou égal à 1,6, en particulier supérieur ou égal à 1,8, plus particulièrement supérieur ou égal à 2,0.

Selon un mode de réalisation préféré, ledit agent d'extraction organique a une capacité d'absorption $C_{2,S}$ supérieure ou égale à 0,20, ladite capacité d'absorption étant calculée par la formule $C_{2,S} = 1/(\gamma_{2,S})$ dans laquelle $\gamma_{2,S}$ représente le coefficient d'activité du trans-1,3,3,3-tetrafluoro-1-propene (1234ze-E) dans ledit agent d'extraction organique à dilution infinie. Avantageusement, la capacité d'absorption $C_{2,S}$ est supérieure ou égale à 0,40, de préférence supérieure ou égale à 0,60, plus préférentiellement supérieure ou égale à 0,80, en particulier supérieure ou égale à 1,0.

Selon un mode de réalisation préféré, ledit agent d'extraction organique peut avoir un facteur de séparation $S_{1,2}$ supérieur ou égal à 1,1, avantageusement supérieur ou égal à 1,2, de préférence supérieur ou égal à 1,4, plus préférentiellement supérieur ou égal à 1,6, en particulier supérieur ou égal à 1,8 et plus particulièrement supérieur ou égal à 2,0 ; et une capacité d'absorption $C_{2,S}$ supérieure ou égale à 0,20, avantageusement supérieure ou égale à 0,40, de préférence supérieure ou égale à 0,60, plus préférentiellement supérieure ou égale à 0,80, en particulier supérieure ou égale à 1,0.

Le trans-1,3,3,3-tetrafluoro-1-propene (1234ze-E) étant une des principales impuretés à éliminer, le facteur de séparation et ladite capacité d'absorption peuvent être calculés pour le couple binaire particulier consistant en 2,3,3,3-tetrafluoro-1-propène et trans-1,3,3,3-tetrafluoro-1-propene (1234ze-E) et/ou 1,1,1,2,2-pentafluoropropane (245cb) et trans-1,3,3,3-tetrafluoro-1-propene (1234ze-E). Le facteur de séparation $S_{1,2}$ permet de déterminer la capacité d'un agent d'extraction organique à séparer deux ou plusieurs composés. La capacité d'absorption $C_{2,S}$ permet de déterminer la quantité de solvant à utiliser pour obtenir la séparation entre les composés considérés.

Ledit agent d'extraction organique peut avoir un facteur de séparation $S_{1,2}$ supérieur ou égal à 1,4, ledit facteur de séparation étant calculé par la formule $S_{1,2} = (\gamma_{1,3}*P1)/(\gamma_{2,3}*P2)$ dans laquelle $\gamma_{1,S}$ représente le coefficient d'activité du 2,3,3,3-tetrafluoro-1-propène par rapport audit agent d'extraction organique, P1 représente la pression de vapeur saturante du 2,3,3,3-tetrafluoro-1-propène, $\gamma_{2,S}$ représente le coefficient d'activité du trans-1,3,3,3-tetrafluoro-1-propene dans ledit agent d'extraction organique à dilution infinie, P2 représente la pression de vapeur saturante du trans-1,3,3,3-tetrafluoro-1-propene et/ou ledit agent d'extraction organique peut avoir un facteur de séparation $S_{1,2}$ supérieur ou égal à 1,4, ledit facteur de séparation étant calculé par la formule $S_{1,2} = (\gamma_{1,3}*P1)/(\gamma_{2,3}*P2)$ dans laquelle $\gamma_{1,S}$ représente le coefficient d'activité du 1,1,1,2,2-pentafluoropropane (245cb) par rapport audit agent d'extraction organique, P1 représente la pression de vapeur saturante du 1,1,1,2,2-pentafluoropropane (245cb), $\gamma_{2,S}$ représente le coefficient d'activité du trans-1,3,3,3-tetrafluoro-1-propene dans ledit agent d'extraction organique à dilution infinie, P2 représente la pression de vapeur saturante du trans-1,3,3,3-tetrafluoro-1-propene ; et de préférence ledit agent d'extraction organique peut avoir une capacité d'absorption $C_{2,S}$ supérieure ou égale à 0,60, ladite capacité d'absorption étant calculé par la formule $C_{2,S} = 1/(\gamma_{2,S})$ dans laquelle $\gamma_{2,S}$ représente le coefficient d'activité de trans-1,3,3,3-tetrafluoro-1-propene dans ledit agent d'extraction organique à dilution infinie ; ledit agent d'extraction organique peut ainsi être éthylamine, acetaldehyde, isopropylamine, methylformate, diethylether, 1,2-epoxypropane, ethylmethylamine, dimethoxymethane, 2-amino-2-methylpropane, methylcyclopropylether, n-propylamine, isopropylmethylamine, 2-ethoxy-propane, methyl-t-butylether, diethylamine, propanone, methylacetate, 4-methoxy-2-methyl-2-butanethiol, 2-butanamine, n-methylpropylamine, isobutanal, tetrahydrofurane, isopropylformate, diisopropylether, 2-ethoxy-2-methyl-propane, 1-butylamine, ethylacetate, butanone, n-propylformate, 2-ethoxy-butane, 1-methoxy-2-methyl-butane, 2,2-dimethoxypropane, 1-ethoxy-2-methyl-propane, diisopropylamine, 1,2-dimethoxyethane, 3-methyl-2-butanamine, diethoxymethane, isopropylacetate, di-n-propylether, 3-pentylamine, n-methylbutylamine, diethylsulfide, 1-ethoxybutane, 1-methoxy-2-propanamine, 2-methylbutanal, 2-methoxyethanamine, tert-butylacetate, propionitrile, 2-allyloxyethanol, 1-methoxy-pentane, ethylpropionate, 1,2-dimethoxypropane, isopropyl-isobutyl-ether, dioxane, 3-pentanone, 1,1-diethoxyethane, 2-pentanone, 2-methoxy-1propanamine, trimethoxymethane, n-pentylamine, 3,3-dimethyl-2-butanone, 1,3-dioxane, piperidine, dipropylamine, 2-ethoxyethanamine, sec-butylacetate, n-methyl-1,2-ethanediamine, 2,2-diethoxypropane, pyridine, n-methylmorpholine, 4-methyl-2-pentanone, 1,2-diaminoethane, isobutyl-tert-butylether, butyronitrile, sec-butyl-tert-butylether, 1-methoxy2-propanol, 1,2-propanediamine, 2,6-dimethyl-5-heptenal, 1-(dimethylamino)-2-propanol, 3-methyl-3-pentanol, 1,1-diethoxypropane, 2-ethylbutylamine, diethylcarbonate, n-butylacetate, 2-hexanone, n-ethylethylenediamine, 5-hexen-2-one, 1,1-diethoxy-n,n-dimethylmethanamine, 2-methylpyridine, 2-methoxy1-propanol, hexanal, 1-ethoxy-2-propanol, 4-methyl-2-hexanamine, hexylamine, methoxycyclohexane, 2-(dimethylamino)-ethanol, cyclohexylamine, n-ethyl-2-dimethylaminoethylamine, ethoxyethanol, 2-methylpyrazine, 2-ethoxy-1-propanol, n-ethyl-morpholine, 1-methylpiperazine, 1,3-propanediamine, di-n-butylether, valeronitrile, 2-heptanamine, 1-ethoxy-hexane, n,n-diethylethylenediamine, 2,6-dimethylpyridine, 4-methyl-2-hexanone, 1,1,1-triethoxyethane, 1-methoxy-2-acetoxypropane, 4-methylpyridine, n,n'-diethyl-1,2-ethanediamine, 2,6-dimethylmorpholine, methylhexanoate, 2-propoxyethanol, 1-propoxy-2-propanol.

Ledit agent d'extraction organique peut avoir un facteur de séparation $S_{1,2}$ supérieur ou égal à 1,6, ledit facteur de séparation étant calculé par la formule $S_{1,2} = (\gamma_{1,S}*P1)/(\gamma_{2,S}*P2)$ dans laquelle $\gamma_{1,S}$ représente le coefficient d'activité du 2,3,3,3-tetrafluoro-1-propène par rapport audit agent d'extraction organique, P1 représente la pression de vapeur saturante du 2,3,3,3-tetrafluoro-1-propène, $\gamma_{2,S}$ représente le coefficient d'activité du trans-1,3,3,3-tetrafluoro-1-propene dans ledit agent d'extraction organique à dilution infinie, P2 représente la pression de vapeur saturante du trans-1,3,3,3-tetrafluoro-1-propene et/ou ledit agent d'extraction organique peut avoir un facteur de séparation $S_{1,2}$ supérieur ou égal à 1,6, ledit facteur de séparation étant calculé par la formule $S_{1,2} = (\gamma_{1,S}*P1)/(\gamma_{2,S}*P2)$ dans laquelle $\gamma_{1,S}$ représente le coefficient d'activité du 1,1,1,2,2-pentafluoropropane (245cb) par rapport audit agent d'extraction organique, P1 représente la pression de vapeur saturante du 1,1,1,2,2-pentafluoropropane (245cb), $\gamma_{2,S}$ représente le coefficient d'activité du trans-1,3,3,3-tetrafluoro-1-propene dans ledit agent d'extraction organique à dilution infinie, P2 représente la pression de vapeur saturante du trans-1,3,3,3-tetrafluoro-1-propene ; et de préférence ledit agent d'extraction organique peut avoir une capacité d'absorption $C_{2,S}$ supérieure ou égale à 0,80, ladite capacité d'absorption étant calculé par la formule $C_{2,S} = 1/(yz,s)$ dans laquelle $\gamma_{2,S}$ représente le coefficient d'activité de trans-1,3,3,3-tetrafluoro-1-propene dans ledit agent d'extraction organique à dilution infinie ; ledit agent d'extraction organique peut ainsi être éthylamine, isopropylamine, diethylether, ethylmethylamine, 2-amino-2-methylpropane, n-propylamine, isopropylmethylamine, 2-ethoxypropane, methyl-t-butylether, diethylamine, propanone, methylacetate, 2-butanamine, n-methylpropylamine, isobutanal, tetrahydrofurane, 1-butylamine, ethylacetate, butanone, n-propylformate, 2,2-dimethoxypropane, 1-ethoxy-2-methylpropane, 1,2-dimethoxyethane, 3-methyl-2-butanamine, diethoxymethane, isopropylacetate, 3-pentylamine, n-methylbutylamine, 1-ethoxybutane, 1-methoxy-2-propanamine, 2-methylbutanal, 2-methoxyethanamine, tert-butylacetate, 1-methoxy-pentane, ethylpropionate, 1,2-dimethoxypropane, dioxane, 3-pentanone, 1,1-diethoxyethane, 2-pentanone, 2-methoxy-1propanamine, trimethoxymethane, n-pentylamine, 3,3-dimethyl-2-butanone, 1,3-dioxane, piperidine, 2-ethoxyethanamine, sec-butylacetate, n-methyl-1,2-ethanediamine, 2,2-diethoxypropane, 4-methyl-2-pentanone, 1,2-diaminoethane, butyronitrile, 1-methoxy2-propanol, 1,2-propanediamine, 2,6-dimethyl-5-heptenal, 1-(dimethylamino)-2-propanol, 1,1-diethoxypropane, 2-ethylbutylamine, diethylcarbonate, n-butylacetate, 2-hexanone, n-ethylethylenediamine, 5-hexen-2-one, 2-methylpyridine, 2-methoxy1-propanol, hexanal, 1-ethoxy-2-propanol, 4-methyl-2, hexanamine, hexylamine, methoxycyclohexane, 2-(dimethylamino)-ethanol, cyclohexylamine, n-ethyl-2-dimethylaminoethylamine, 2-methylpyrazine, 2-ethoxy-1-propanol, 1-methylpiperazine, 1,3-propanediamine, di-n-butylether, valeronitrile, 2-heptanamine, 1-ethoxy-hexane, n,n-diethylethylenediamine, 2,6-dimethylpyridine, 4-methyl-2-hexanone, 1,1,1-triethoxyethane, 1-methoxy-2-acetoxypropane, 4-methylpyridine, n,n'-diethyl-1,2-ethanediamine, 2,6-dimethylmorpholine, methylhexanoate, 2-propoxyethanol, 1-propoxy-2-propanol.

De préférence, ledit agent d'extraction organique peut être éthylamine, isopropylamine, diethylether, ethylmethylamine, 2-amino-2-methylpropane, n-propylamine, isopropylmethylamine, 2-ethoxy-propane, methyl-t-butylether, diethylamine, propanone, methylacetate, 2-butanamine, n-methylpropylamine, isobutanal, tetrahydrofurane, 1-butylamine, ethylacetate, butanone, n-propylformate, 2,2-dimethoxypropane, 1-ethoxy-2-methylpropane, 1,2-dimethoxyethane, 3-methyl-2-butanamine, diethoxymethane, isopropylacetate, 3-pentylamine, n-methylbutylamine, 1-ethoxybutane, 1-methoxy-2-propanamine, 2-methylbutanal, 2-methoxyethanamine, tert-butylacetate, 1-methoxy-pentane, ethylpropionate, 1,2-dimethoxypropane, dioxane, 3-pentanone, 1,1-diethoxyethane, 2-pentanone, 2-methoxy-1propanamine, trimethoxymethane, n-pentylamine, 3,3-dimethyl-2-butanone, 1,3-dioxane, piperidine, 2-ethoxyethanamine, sec-butylacetate, n-methyl-1,2-ethanediamine, 2,2-diethoxypropane, 4-methyl-2-pentanone, 1,2-diaminoethane, butyronitrile, 1-methoxy2-propanol, 1,2-propanediamine, 2,6-dimethyl-5-heptenal, 1-(dimethylamino)-2-propanol, 1,1-diethoxypropane, 2-ethylbutylamine, diethylcarbonate, n-butylacetate, 2-hexanone, n-ethylethylenediamine, 5-hexen-2-one, 2-methylpyridine, 2-methoxy1-propanol, hexanal. En particulier, ledit agent d'extraction organique peut être éthylamine, isopropylamine, diethylether, ethylmethylamine, 2-amino-2-methylpropane, n-propylamine, isopropylmethylamine, 2-ethoxypropane, diethylamine, 2-butanamine, n-methylpropylamine, 1-butylamine, n-propylformate, 2,2-dimethoxypropane, 1-ethoxy-2-methylpropane, 3-methyl-2-butanamine, diethoxymethane, isopropylacetate, 3-pentylamine, n-methylbutylamine, 1-ethoxybutane, 1-methoxy-2-propanamine, 2-methylbutanal, 2-methoxyethanamine, tert-butylacetate, 1-methoxy-pentane, 1,2-dimethoxypropane, dioxane, ,1-diethoxyethane, 2-methoxy-1propanamine, trimethoxymethane, n-pentylamine, 1,3-dioxane, piperidine, 2-ethoxyethanamine, sec-butylacetate, n-methyl-1,2-ethanediamine, 2,2-diethoxypropane, 1,2-diaminoethane, butyronitrile, 1-methoxy2-propanol, 1,2-propanediamine, 2,6-dimethyl-5-heptenal, 1-(dimethylamino)-2-propanol, 1,1-diethoxypropane, 2-ethylbutylamine, n-butylacetate, n-ethylethylenediamine, 5-hexen-2-one, 2-methylpyridine, 2-methoxy1-propanol, hexanal.

Selon un mode de réalisation préféré, ledit agent d'extraction organique peut être éthylamine, isopropylamine, diethylether, n-propylamine, diethylamine, propanone, methylacetate, butanone, diethoxymethane, isopropylacetate, 3-pentylamine, 2-methoxyethanamine, tert-butylacetate, dioxane, 1,1-diethoxyethane, trimethoxymethane, n-pentylamine, 1,3-dioxane, sec-butylacetate, 1,2-diaminoethane, 1-methoxy-2-propanol, 1,2-propanediamine, n-butylacetate, 2-methoxy-1-propanol, hexanal. En particulier, ledit agent d'extraction organique peut être éthylamine, isopropylamine, diethylether, n-propylamine, diethylamine, diethoxymethane, isopropylacetate, 3-pentylamine, 2-methoxyethanamine, tert-butylacetate, dioxane, 1,1-diethoxyethane, trimethoxymethane, n-pentylamine, 1,3-dioxane, sec-butylacetate, 1,2-diaminoethane, 1-methoxy-2-propanol, 1,2-propanediamine, n-butylacetate, 2-methoxy-1-propanol, hexanal.

Selon un mode de réalisation préféré, ladite troisième composition comprenant ledit agent d'extraction organique et trans-1,3,3,3-tétrafluoro-1-propène (1234ze-E); de préférence la troisième composition comprenant ledit agent d'extraction organique et trans-1,3,3,3-tétrafluoro-1-propène (1234ze-E) et optionnellement ou non au moins un des composés choisi parmi le groupe consistant en chlorométhane (40), 1,1-difluoroéthane (152a), 1,1,1,2-tétrafluoroethane (134a), chloropentafluoroéthane (115), trans-1,2,3,3,3-pentafluoropropène (1225ye-E), cis-1,2,3,3,3-pentafluoropropène (1225ye-Z), 3,3,3-trifluoropropène (1243zf) ; peut être soumise à une distillation pour séparer l'agent d'extraction organique et le trans-1,3,3,3-tétrafluoro-1-propène (1234ze-E); de préférence pour séparer d'une part l'agent d'extraction organique et d'autre part le trans-1,3,3,3-tétrafluoro-1-propène (1234ze-E) et optionnellement ou non au moins un des composés choisi parmi le groupe consistant en chlorométhane (40), 1,1-difluoroéthane (152a), 1,1,1,2-tétrafluoroethane (134a), chloropentafluoroéthane (115), trans-1,2,3,3,3-pentafluoropropène (1225ye-E), cis-1,2,3,3,3-pentafluoropropène (1225ye-Z), 3,3,3-trifluoropropène (1243zf). Ledit agent d'extraction organique est ainsi recyclé à l'étape a) du procédé de purification. Le courant comprenant le trans-1,3,3,3-tétrafluoro-1-propène (1234ze-E) et optionnellement ou non au moins un des composés choisi parmi le groupe consistant en chlorométhane (40), 1,1-difluoroéthane (152a), 1,1,1,2-tétrafluoroethane (134a), chloropentafluoroéthane (115), trans-1,2,3,3,3-pentafluoropropène (1225ye-E), cis-1,2,3,3,3-pentafluoropropène (1225ye-Z), 3,3,3-trifluoropropène (1243zf) peut être soit soumis à une ou plusieurs étapes de purification ultérieures ou peut être détruit par incinération.

Le présent procédé permet donc de purifier le 2,3,3,3-tétrafluoro-1-propène (1234yf). Avantageusement, la teneur en trans-1,3,3,3-tétrafluoro-1-propène (1234ze-E) dans le courant comprenant du 2,3,3,3-tétrafluoro-1-propène (1234yf) et 1,1,1,2,2-pentafluoropropane (245cb) et obtenu à l'étape b) du présent procédé de purification est inférieure à la teneur de celui-ci dans ladite première composition. De préférence, la teneur en trans-1,3,3,3-tétrafluoro-1-propène (1234ze-E) et/ou en au moins un des composés sélectionné parmi le groupe consistant en chlorométhane (40), 1,1-difluoroéthane (152a), 1,1,1,2-tétrafluoroethane (134a), chloropentafluoroéthane (115), trans-1,2,3,3,3-pentafluoropropène (1225ye-E), cis-1,2,3,3,3-pentafluoropropène (1225ye-Z), 3,3,3-trifluoropropène (1243zf) dans le courant comprenant du 2,3,3,3-tétrafluoro-1-propène (1234yf) et 1,1,1,2,2-pentafluoropropane (245cb) et obtenu à l'étape b) du présent procédé de purification est inférieure à la teneur de celui-ci dans ladite première composition.

Par exemple, la teneur en trans-1,3,3,3-tétrafluoro-1-propène (1234ze-E) ou en l'un quelconque des composés sélectionnés parmi le groupe consistant en chlorométhane (40), 1,1-difluoroéthane (152a), 1,1,1,2-tétrafluoroethane (134a), chloropentafluoroéthane (115), trans-1,2,3,3,3-pentafluoropropène (1225ye-E), cis-1,2,3,3,3-pentafluoropropène (1225ye-Z), 3,3,3-trifluoropropène (1243zf) peut être diminuée de 50%, avantageusement de 75%, de préférence de 90%, en particulier de 95%, plus particulièrement de 98%. Avantageusement, la teneur en au moins deux, au moins trois, au moins quatre, au moins cinq, au moins six ou en l'ensemble desdits composés sélectionné parmi le groupe consistant en chlorométhane (40), 1,1-difluoroéthane (152a), 1,1,1,2-tétrafluoroethane (134a), chloropentafluoroéthane (115), trans-1,2,3,3,3-pentafluoropropène (1225ye-E), cis-1,2,3,3,3-pentafluoropropène (1225ye-Z), 3,3,3-trifluoropropène (1243zf) peut être diminuée de 50%, avantageusement de 75%, de préférence de 90%, en particulier de 95%, plus particulièrement de 98%. De préférence, la teneur en trans-1,3,3,3-tétrafluoro-1-propène (1234ze-E) peut être diminuée de 50%, avantageusement de 75%, de préférence de 90%, en particulier de 95%, plus particulièrement de 98%.

De préférence, le courant comprenant le 2,3,3,3-tétrafluoro-1-propène et 1,1,1,2,2-pentafluoropropane obtenu à l'étape b) du présent procédé de purification peut être dépourvu de trans-1,3,3,3-tétrafluoro-1-propène (1234ze-E). En particulier, le courant comprenant le 2,3,3,3-tétrafluoro-1-propène et 1,1,1,2,2-pentafluoropropane obtenu à l'étape b) du présent procédé de purification peut être dépourvu de trans-1,3,3,3-tétrafluoro-1-propene (1234ze-E) et/ou optionnellement d'au moins un, d'au moins deux, d'au moins trois, d'au moins quatre, d'au moins cinq, d'au moins six ou de l'ensemble des composés sélectionné parmi le groupe consistant en chlorométhane (40), 1,1-difluoroéthane (152a), 1,1,1,2-tétrafluoroethane (134a), chloropentafluoroéthane (115), trans-1,2,3,3,3-pentafluoropropène (1225ye-E), cis-1,2,3,3,3-pentafluoropropène (1225ye-Z), 3,3,3-trifluoropropène (1243zf) lorsque celui-ci ou ceux-ci sont présents dans ladite première composition. Le terme « dépourvu » signifie que le courant comprenant le 2,3,3,3-tétrafluoro-1-propène (1234yf) comprend moins de 50 ppm, avantageusement moins de 20 ppm, de préférence moins de 10 ppm du composé considéré sur base du poids total du courant. Les teneurs sont exprimées en poids.

Selon un mode de réalisation préféré, ladite première composition mise en oeuvre à l'étape a) du présent procédé peut être préalablement purifiée avant d'être utilisée. En effet, si ladite première composition comprend des impuretés ayant un point d'ébullition inférieur au point d'ébullition du 2,3,3,3-tétrafluoro-1-propène et optionnellement des impuretés lourdes, le présent procédé peut comprendre préalablement à l'étape a) les étapes :

i') mise en oeuvre d'une composition comprenant 2,3,3,3-tétrafluoro-1-propène, des impuretés ayant un point d'ébullition inférieur au point d'ébullition du 2,3,3,3-tétrafluoro-1-propène, 1,1,1,2,2-pentafluoropropane (245cb), et trans-1,3,3,3-tétrafluoro-1-propene (1234ze-E), optionnellement ou non au moins un des composés sélectionnés parmi le groupe consistant en chlorométhane (40), 1,1-difluoroéthane (152a), 1,1,1,2-tétrafluoroethane (134a), chloropentafluoroéthane (115), trans-1,2,3,3,3-pentafluoropropène (1225ye-E), cis-1,2,3,3,3-pentafluoropropene (1225ye-Z), 3,3,3-trifluoropropene (1243zf), et optionnellement ou non des impuretés lourdes ;

ii') distillation de ladite composition de l'étape i) pour éliminer en tête de colonne des impuretés ayant un point d'ébullition inférieur au point d'ébullition du 2,3,3,3-tetrafluoro-1-propène et former un premier courant comprenant 2,3,3,3-tetrafluoro-1-propène, 1,1,1,2,2-pentafluoropropane (245cb), et trans-1,3,3,3-tetrafluoro-1-propene (1234ze-E), optionnellement ou non au moins un des composés sélectionnés parmi le groupe consistant en chlorométhane (40), 1,1-difluoroéthane (152a), 1,1,1,2-tetrafluoroethane (134a), chloropentafluoroéthane (115), trans-1,2,3,3,3-pentafluoropropene (1225ye-E), cis-1,2,3,3,3-pentafluoropropene (1225ye-Z), 3,3,3-trifluoropropene (1243zf), et optionnellement ou non des impuretés lourdes, récupéré en bas de colonne de distillation ;

iii') optionnellement ou non, distillation dudit premier courant récupéré en bas de colonne de distillation à l'étape ii') pour récupérer en tête de colonne un second courant comprenant du 2,3,3,3-tetrafluoro-1-propène, 1,1,1,2,2-pentafluoropropane (245cb), et trans-1,3,3,3-tetrafluoro-1-propene (1234ze-E), optionnellement ou non au moins un des composés sélectionnés parmi le groupe consistant en chlorométhane (40), 1,1-difluoroéthane (152a), 1,1,1,2-tetrafluoroethane (134a), chloropentafluoroéthane (115), trans-1,2,3,3,3-pentafluoropropene (1225ye-E), cis-1,2,3,3,3-pentafluoropropene (1225ye-Z), 3,3,3-trifluoropropene (1243zf), et en bas de colonne de distillation un courant comprenant les impuretés lourdes ; ledit premier courant récupéré à l'étape ii') ou ledit second courant récupéré à l'étape

iii') correspondent à ladite première composition mise en oeuvre à l'étape a).

Selon un second aspect, la présente invention fournit un procédé de production du 2,3,3,3-tetrafluoro-1-propène. En outre, ce procédé peut inclure sa purification. Ainsi, la présente invention fournit un procédé de production de 2,3,3,3-tetrafluoro-1-propène comprenant les étapes de :

A) fluoration en présence d'un catalyseur d'un composé de formule (I) $CX(Y)_2-CX(Y)_m-CH_mXY$ dans laquelle X et Y représentent indépendamment un atome d'hydrogène, de fluor ou de chlore et m = 0 ou 1 ; et/ou fluoration catalytique en présence d'un catalyseur d'un composé de formule $(CX_nY_{3-n})CH_pX_{1-p}CH_mX_{2-m}$ (II) dans lequel X est indépendamment les uns des autres Cl, F, I ou Br ; Y est indépendamment les uns des autres H, Cl, F, I ou Br ; n est 1, 2 ou 3 ; et m est 0, 1 ou 2 ; et p est 0 ou 1 ;

B) récupération d'un courant comprenant du 2,3,3,3-tetrafluoro-1-propène, 1,1,1,2,2-pentafluoropropane (245cb), trans-1,3,3,3-tetrafluoro-1-propene (1234ze-E), et optionnellement ou non au moins un des composés sélectionnés parmi le groupe consistant en chlorométhane (40), 1,1-difluoroéthane (152a), 1,1,1,2-tetrafluoroethane (134a), chloropentafluoroéthane (115), trans-1,2,3,3,3-pentafluoropropene (1225ye-E), cis-1,2,3,3,3-pentafluoropropene (1225ye-Z), 3,3,3-trifluoropropene (1243zf) ;

C) mise en oeuvre du procédé de purification du 2,3,3,3-tetrafluoro-1-propène selon la présente invention à partir du courant récupéré à l'étape B).

Selon un mode de réalisation préféré, le procédé de production de 2,3,3,3-tetrafluoro-1-propène comprenant les étapes de :

A) fluoration en présence d'un catalyseur d'un composé de formule (I) $CX(Y)_2-CX(Y)_m-CH_mXY$ dans laquelle X et Y représentent indépendamment un atome d'hydrogène, de fluor ou de chlore et m = 0 ou 1 ; et/ou fluoration catalytique en présence d'un catalyseur d'un composé de formule $(CX_nY_{3-n})CH_pX_{1-p}CH_mX_{2-m}$ (II) dans lequel X est indépendamment les uns des autres Cl, F, I ou Br ; Y est indépendamment les uns des autres H, Cl, F, I ou Br ; n est 1, 2 ou 3 ; et m est 0, 1 ou 2 ; et p est 0 ou 1 ;

B) récupération d'un courant comprenant du 2,3,3,3-tetrafluoro-1-propène, 1,1,1,2,2-pentafluoropropane (245cb), trans-1,3,3,3-tetrafluoro-1-propene (1234ze-E) et optionnellement ou non au moins un des composés choisis parmi le groupe consistant en chlorométhane (40), 1,1-difluoroéthane (152a), 1,1,1,2-tetrafluoroethane (134a), chloropentafluoroéthane (115), trans-1,2,3,3,3-pentafluoropropene (1225ye-E), cis-1,2,3,3,3-pentafluoropropene (1225ye-Z), 3,3,3-trifluoropropene (1243zf);

C) mise en oeuvre du procédé de purification du 2,3,3,3-tetrafluoro-1-propène à partir du courant récupéré à l'étape B) et comprenant les étapes de :

a) mise en contact du courant récupéré à l'étape B) avec au moins un agent d'extraction organique pour former une seconde composition ;

b) distillation extractive de ladite seconde composition pour former :

i) une troisième composition comprenant ledit agent d'extraction organique, trans-1,3,3,3-tetrafluoro-1-propene (1234ze-E) et optionnellement ou non ledit au moins un des composés sélectionné parmi le groupe consistant en chlorométhane (40), 1,1-difluoroéthane (152a), 1,1,1,2-tetrafluoroethane (134a), chloropentafluoroéthane (115), trans-1,2,3,3,3-pentafluoropropene (1225ye-E), cis-1,2,3,3,3-pentafluoropropene (1225ye-Z), 3,3,3-trifluoropropene (1243zf); et

ii) un courant comprenant le 2,3,3,3-tétrafluoro-1-propène et 1,1,1,2,2-pentafluoropropane,

c) récupération et séparation de ladite troisième composition pour former d'une part un courant comprenant ledit agent d'extraction organique et d'autre part un courant comprenant trans-1,3,3,3-tetrafluoro-1-propene (1234ze-E) et optionnellement ou non au moins un des composés sélectionné parmi le groupe consistant en chlorométhane (40), 1,1-difluoroéthane (152a), 1,1,1,2-tetrafluoroethane (134a), chloropentafluoroéthane (115), trans-1,2,3,3,3-pentafluoropropene (1225ye-E), cis-1,2,3,3,3-pentafluoropropene (1225ye-Z), 3,3,3-trifluoropropene (1243zf).

De préférence, le courant comprenant ledit agent d'extraction organique peut être recyclé à l'étape a). De préférence, le courant comprenant trans-1,3,3,3-tetrafluoro-1-propene (1234ze-E) et optionnellement ou non au moins un des composés sélectionné parmi le groupe consistant en chlorométhane (40), 1,1-difluoroéthane (152a), 1,1,1,2-tetrafluoroethane (134a), chloropentafluoroéthane (115), trans-1,2,3,3,3-pentafluoropropene (1225ye-E), cis-1,2,3,3,3-pentafluoropropene (1225ye-Z), 3,3,3-trifluoropropene (1243zf) peut être purifié ou détruit par incinération.

Selon un mode de réalisation particulier, le courant récupéré à l'étape B) du procédé de production du 2,3,3,3-tétrafluoro-1-propène peut comprendre de l'acide fluorhydrique. Dans ce cas, préalablement à l'étape C), le courant récupéré à l'étape B) peut subir une distillation préalable B1') pour éliminer HF en bas de colonne de distillation. Un courant comprenant 2,3,3,3-tétrafluoro-1-propène, 1,1,1,2,2-pentafluoropropane, trans-1,3,3,3-tetrafluoro-1-propene (1234ze-E) et optionnellement ou non au moins un des composés choisi parmi le groupe consistant en chlorométhane (40), 1,1-difluoroéthane (152a), 1,1,1,2-tetrafluoroethane (134a), chloropentafluoroéthane (115), trans-1,2,3,3,3-pentafluoropropene (1225ye-E), cis-1,2,3,3,3-pentafluoropropene (1225ye-Z), 3,3,3-trifluoropropene (1243zf), est récupéré en tête de colonne de distillation. Ce dernier courant récupéré en tête de colonne de distillation est ensuite soumis à l'étape C) du procédé de production du 2,3,3,3-tetrafluoro-1-propène.

Selon un mode de réalisation particulier, le courant récupéré à l'étape B) du procédé de production du 2,3,3,3-tétrafluoro-1-propène peut comprendre des impuretés ayant un point d'ébullition inférieur au point d'ébullition du 2,3,3,3-tetrafluoro-1-propène. Dans ce cas, préalablement à l'étape C), le courant récupéré à l'étape B) peut subir une distillation préalable B2'), subséquente ou non à l'étape B1'), pour éliminer en tête de colonne de distillation lesdites impuretés ayant un point d'ébullition inférieur au point d'ébullition du 2,3,3,3-tetrafluoro-1-propène et former un premier courant comprenant 2,3,3,3-tetrafluoro-1-propène, 1,1,1,2,2-pentafluoropropane, trans-1,3,3,3-tetrafluoro-1-propene (1234ze-E) et optionnellement ou non au moins un des composés choisi parmi le groupe consistant en chlorométhane (40), 1,1-difluoroéthane (152a), 1,1,1,2-tetrafluoroethane (134a), chloropentafluoroéthane (115), trans-1,2,3,3,3-pentafluoropropene (1225ye-E), cis-1,2,3,3,3-pentafluoropropene (1225ye-Z), 3,3,3-trifluoropropene (1243zf), récupéré en bas de colonne de distillation. Ce dernier courant récupéré en bas de colonne de distillation est ensuite soumis à l'étape C) du procédé de production du 2,3,3,3-tetrafluoro-1-propène.

Selon un mode de réalisation particulier, le courant récupéré à l'étape B) du procédé de production du 2,3,3,3-tétrafluoro-1-propène peut comprendre des impuretés lourdes. Dans ce cas, préalablement à l'étape C), le courant récupéré à l'étape B) peut subir une distillation préalable B3'), subséquente ou non à l'étape B1') et/ou B2'), pour éliminer en bas de colonne de distillation lesdites impuretés lourdes ; et former un premier courant comprenant 2,3,3,3-tetrafluoro-1-propène, 1,1,1,2,2-pentafluoropropane, trans-1,3,3,3-tetrafluoro-1-propene (1234ze-E) et optionnellement ou non au moins un des composés choisi parmi le groupe consistant en chlorométhane (40), 1,1-difluoroéthane (152a), 1,1,1,2-tetrafluoroethane (134a), chloropentafluoroéthane (115), trans-1,2,3,3,3-pentafluoropropene (1225ye-E), cis-1,2,3,3,3-pentafluoropropene (1225ye-Z), 3,3,3-trifluoropropene (1243zf), récupéré en tête de colonne de distillation. Ce dernier courant récupéré en tête de colonne de distillation est ensuite soumis à l'étape C) du procédé de production du 2,3,3,3-tetrafluoro-1-propène.

Selon un mode de réalisation particulier, le courant récupéré à l'étape B) du procédé de production du 2,3,3,3-tétrafluoro-1-propène peut également comprendre HCl. L'acide chlorhydrique peut être récupéré par distillation avant ou après l'étape B1' indépendamment des autres étapes du procédé.

Plus particulièrement, l'étape A) est effectuée à partir de 1,1,2,3-tetrachloropropène, le 2,3,3,3,-tetrachloropropène, le 1,1,3,3-tetrachloropropène, le 1,3,3,3-tetrachloropropène, le 1,1,1,2,3-pentachloropropane, le 1,1,1,3,3-pentachloropropane, le 1,1,2,2,3-pentachloropropane, le 1,2-dichloro-3,3,3-trifluoropropane, le 2-chloro-2,3,3,3-tetrafluoropropane, le 1,1,1,2,2-pentafluoropropane, le 1-chloro-1,3,3,3-tetrafluoropropane et le 1,1,1,3,3-pentafluoropropane, de préférence à partir de 1,1,1,2,3-pentachloropropane, 1,1,2,3,tetrachloropropène, du 1,1,1,2,2-pentafluoropropane et/ou du 2-chloro-3,3,3-trifluoro-1-propène ; en particulier à partir du 1,1,1,2,3-pentachloropropane (240db).

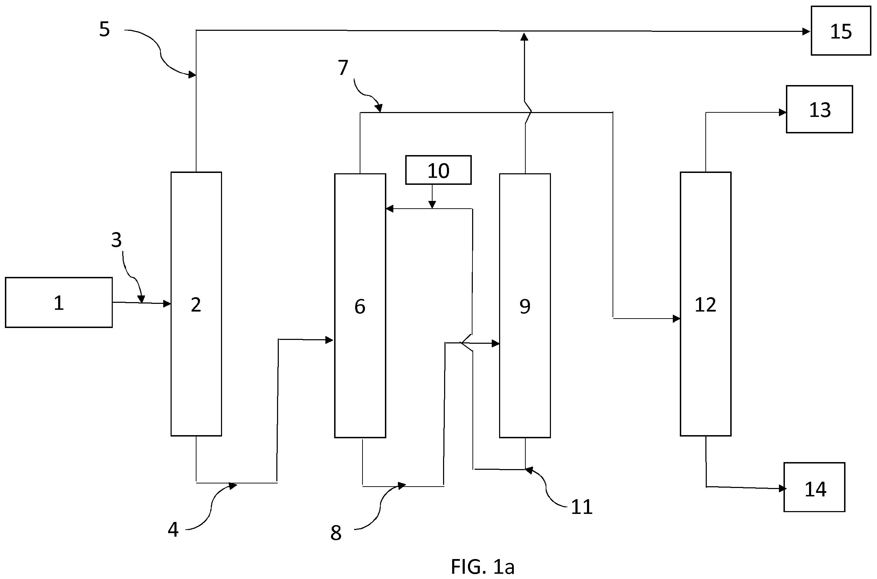

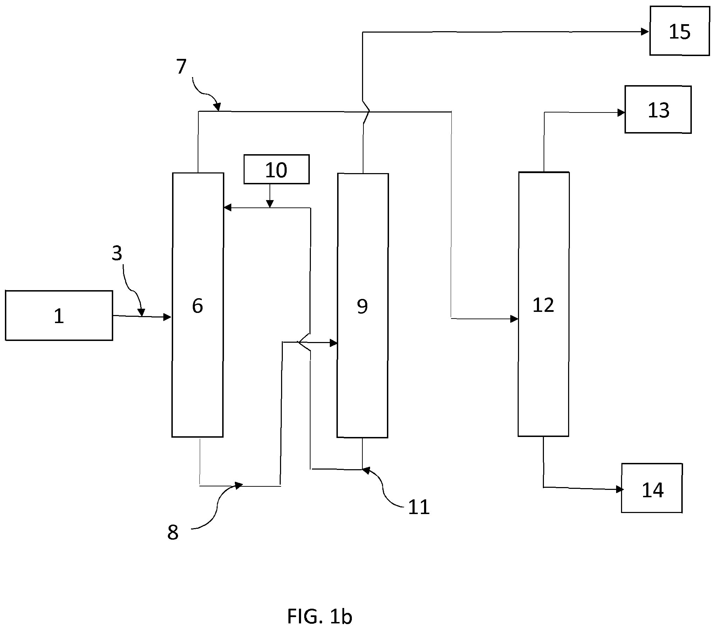

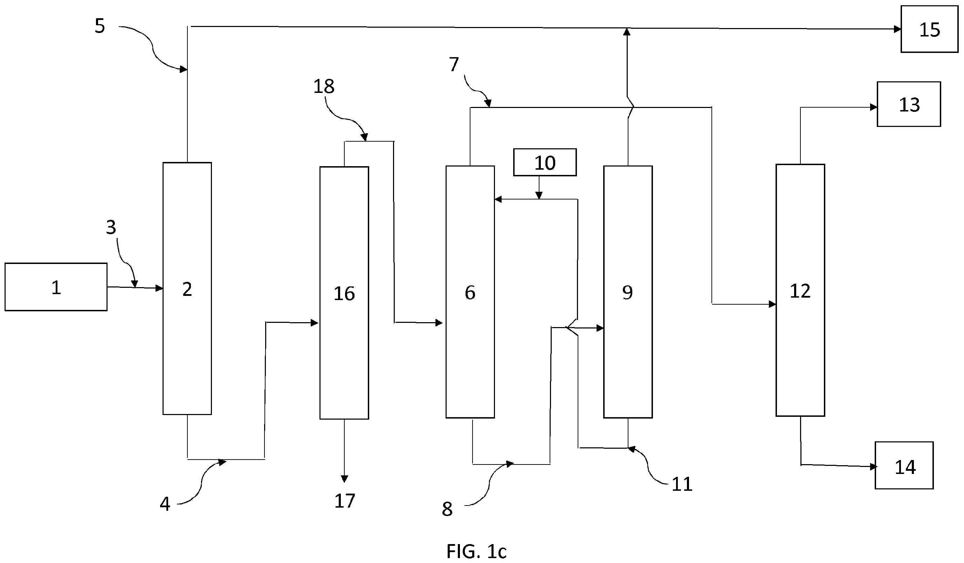

La figure 1 représente un schéma simplifié d'un dispositif mettant en oeuvre un procédé de purification du 2,3,3,3-tetrafluoro-1-propene et du 1,1,1,2,2-pentafluoropropane selon un mode de réalisation particulier de l'invention. Le mélange issu de la réaction de fluoration en présence d'un catalyseur, d'un composé de formule (I)

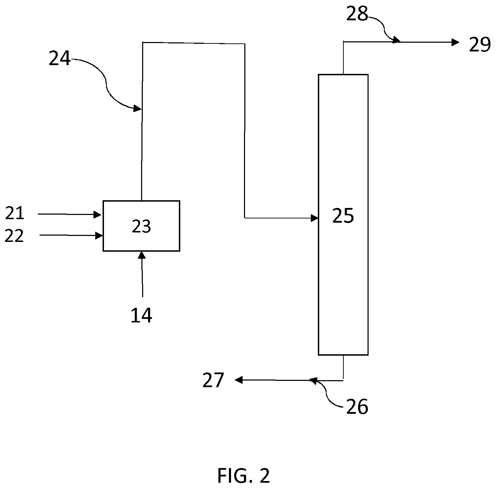

CX(Y)$_2$-CX(Y)$_m$-CH$_m$XY telle que défini dans la présente invention ; et/ou de fluoration catalytique, en présence d'un catalyseur, d'un composé de formule (CX$_n$Y$_{3-n}$)CH$_p$X$_{1-p}$=CH$_m$X$_{2-m}$ (II), telle que défini dans la présente invention est obtenu en 1. Le mélange comprend, dans ce mode de réalisation particulier, 2,3,3,3-tétrafluoro-1-propene, 1,1,1,2,2-pentafluoropropane (245cb), trans-1,3,3,3-tetrafluoro-1-propene (1234ze-E) et des impuretés ayant un point d'ébullition inférieur au point d'ébullition du 2,3,3,3-tetrafluoro-1-propene. Le mélange est transféré dans une colonne de distillation 2 par le conduit 3. Les impuretés ayant un point d'ébullition inférieur au point d'ébullition du 2,3,3,3-tetrafluoro-1-propene sont récupérées en tête de la colonne de distillation 2 et acheminées par le conduit 5 à un incinérateur ou un dispositif de purification 15. Le résidu obtenu en bas de colonne de distillation et comprenant les autres constituants du mélange est acheminé vers une seconde colonne de distillation extractive 6 par le conduit 4. La distillation extractive effectuée en 6 vise à séparer le 2,3,3,3-tetrafluoro-1-propene et le 1,1,1,2,2-pentafluoropropane (245cb) des autres constituants du mélange. La colonne de distillation extractive 6 est approvisionnée avec l'agent d'extraction organique 10. Le courant comprenant 2,3,3,3-tetrafluoro-1-propene et le 1,1,1,2,2-pentafluoropropane (245cb) est récupéré en tête de la colonne de distillation 6 et acheminé vers la colonne de distillation 12 par le conduit 7. L'agent d'extraction organique 10 et le trans-1,3,3,3-tetrafluoro-1-propene (1234ze-E) sont récupérés en bas de la colonne de distillation 6 pour être acheminés vers la colonne de distillation 9 où ceux-ci sont séparés. L'agent d'extraction organique est récupéré en bas de colonne de distillation 9 pour être recyclé vers la colonne de distillation extractive 6 par le conduit 11. Le trans-1,3,3,3-tetrafluoro-1-propene est récupéré en tête de colonne de distillation 9 pour être acheminé vers un incinérateur ou un dispositif de purification 15. La colonne de distillation 12 permet la séparation entre le 2,3,3,3-tetrafluoro-1-propene récupéré en tête de colonne de distillation 13 et le 1,1,1,2,2-pentafluoropropane récupéré en bas de colonne de distillation 14. Le 1,1,1,2,2-pentafluoropropane peut être recyclé dans le réacteur 23 décrit à la Fig. 2 ci-dessous.

Le mélange fourni en 1 peut être dépourvu d'impuretés ayant un point d'ébullition inférieur à celui du 2,3,3,3-tetrafluoro-1-propène. Dans ce cas, comme illustré à la Fig. 1b, le mélange 1 est acheminé par le conduit 3 vers la colonne de distillation extractive 6 pour être traité comme expliqué ci-dessus en relation avec la Fig. 1a. Dans un autre mode de réalisation particulier illustré à la Fig. 1c, le mélange 1 peut comprendre des impuretés lourdes. Dans ce cas, une distillation peut être effectuée à partir du courant obtenu en bas de la colonne de distillation 2. Ce dernier est donc acheminé par le conduit 4 vers la colonne de distillation 16. Les impuretés lourdes sont récupérées en bas de colonne de distillation 17. Le courant récupéré en tête de la colonne de distillation 16 comprend 2,3,3,3-tetrafluoro-1-propene, 1,1,1,2,2-pentafluoropropane (245cb), trans-1,3,3,3-tetrafluoro-1-propene (1234ze-E). Ce courant est transféré par l'intermédiaire du conduit 18 vers la colonne de distillation extractive 6 pour y être traité comme expliqué ci-dessus en relation avec la Fig. 1a. Le mélange obtenu en 1 peut également comprendre au moins un des composés sélectionné parmi le groupe consistant en chlorométhane (40), 1,1-difluoroéthane (152a), 1,1,1,2-tetrafluoroethane (134a), chloropentafluoroéthane (115), trans-1,2,3,3,3-pentafluoropropene (1225ye-E), cis-1,2,3,3,3-pentafluoropropene (1225ye-Z), 3,3,3-trifluoropropene (1243zf). Ces composés se retrouvent dans le même courant que le trans-1,3,3,3-tetrafluoro-1-propene (1234ze-E). Ils sont donc absorbés par l'agent d'extraction organique lors de la distillation extractive 6 pour être acheminés vers la colonne de distillation 9 où ils sont récupérés en tête de colonne de distillation avec le trans-1,3,3,3-tetrafluoro-1-propene (1234ze-E) et sont incinérés ou purifiés en 15.

La Fig. 2 illustre schématiquement un dispositif mettant en oeuvre un procédé de production du 2,3,3,3-tetrafluoropropène selon un mode de réalisation particulier de la présente invention. L'acide fluorhydrique 21 est mis en contact avec le 1,1,1,2,3-pentachloropropane (240db) 22 dans un réacteur 23. Le réacteur 23 peut également être alimenté avec le courant 14 comprenant 1,1,1,2,2-pentafluoropropane (245cb) et provenant du recyclage du procédé de purification du 2,3,3,3-tetrafluoro-1-propène et du 1,1,1,2,2-pentafluoropropane (245cb). Le mélange obtenu et comprenant 2,3,3,3-tetrafluoro-1-propène, 1,1,1,2,2-pentafluoropropane (245cb), trans-1,3,3,3-tetrafluoro-1-propene (1234ze-E) et optionnellement au moins un des composés sélectionné parmi le groupe consistant en chlorométhane (40), 1,1-difluoroéthane (152a), 1,1,1,2-tetrafluoroethane (134a), chloropentafluoroéthane (115), trans-1,2,3,3,3-pentafluoropropene (1225ye-E), cis-1,2,3,3,3-pentafluoropropene (1225ye-Z), 3,3,3-trifluoropropene (1243zf), est récupéré en sortie de réacteur et acheminé vers une colonne de distillation 25 par le conduit 24. Le mélange peut aussi comprendre HCl, HF et des impuretés lourdes ou ayant un point d'ébullition inférieur à celui du 2,3,3,3-tetrafluoro-1-propène. Le courant obtenu en bas de colonne de distillation comprenant HF et éventuellement des impuretés lourdes est acheminé vers le dispositif de purification 27 via le conduit 26 pour purifier HF qui sera recyclé éventuellement en 23. Les autres constituants du mélange sont acheminés via le conduit 28 vers un dispositif 29 de purification du 2,3,3,3-tetrafluoro-1-propène et du 1,1,1,2,2-pentafluoropropane. Le dispositif de purification 29 peut être l'un quelconque des dispositifs illustrés aux Fig. 1a-1c.

Le catalyseur utilisé dans le présent procédé de production de 2,3,3,3-tétrafluoropropène peut être par exemple à base d'un métal comprenant un oxyde de métal de transition ou un dérivé ou un halogénure ou un oxyhalogénure d'un tel métal. On peut citer par exemple FeCl$_3$, l'oxyfluorure de chrome, les oxydes de chrome (éventuellement soumis à des traitements de fluoration), les fluorures de chrome et leurs mélanges. D'autres catalyseurs possibles sont les catalyseurs supportés sur du carbone, les catalyseurs à base d'antimoine, les catalyseurs à base d'aluminium (par exemple AlF$_3$ et Al$_2$O$_3$, l'oxyfluorure d'alumine et le fluorure d'alumine).

On peut utiliser en général un oxyfluorure de chrome, un fluorure ou un oxyfluorure d'aluminium, ou un catalyseur supporté ou non contenant un métal tel que Cr, Ni, Fe, Zn, Ti, V, Zr, Mo, Ge, Sn, Pb, Mg, Sb.

On peut faire référence à cet égard au document WO 2007/079431 (en p.7, l.1-5 et 28-32), au document EP 939071 (paragraphe [0022]), au document WO 2008/054781 (en p.9 l.22-p.10 l.34), et au document WO 2008/040969 (revendication 1), auxquels il est fait expressément référence.

Le catalyseur est de manière plus particulièrement préférée à base de chrome et il s'agit plus particulièrement d'un catalyseur mixte comprenant du chrome.

Selon un mode de réalisation, on utilise un catalyseur mixte comprenant du chrome et du nickel. Le rapport molaire Cr / Ni (sur la base de l'élément métallique) est généralement de 0,5 à 5, par exemple de 0,7 à 2, par exemple d'environ 1. Le catalyseur peut contenir de 0,5 à 20 % en poids de nickel.

Le métal peut être présent sous forme métallique ou sous forme de dérivé, par exemple un oxyde, halogénure ou oxyhalogénure. Ces dérivés sont de préférence obtenus par activation du métal catalytique.

Le support est de préférence constitué avec de l'aluminium, par exemple de l'alumine, de l'alumine activée ou des dérivés d'aluminium, tels que les halogénures d'aluminium et les oxyhalogénures d'aluminium, par exemple décrits dans le document US 4,902,838, ou obtenus par le procédé d'activation décrit ci-dessus.

Le catalyseur peut comprendre du chrome et du nickel sous une forme activée ou non, sur un support qui a été soumis à une activation ou non.

On peut se reporter au document WO 2009/118628 (notamment en p.4, l.30-p.7 l.16), auquel il est fait expressément référence ici.

Un autre mode de réalisation préféré repose sur un catalyseur mixte contenant du chrome et au moins un élément choisi parmi Mg et Zn. Le rapport atomique de Mg ou Zn/Cr est de préférence de 0,01 à 5.

Avant son utilisation, le catalyseur est de préférence soumis à une activation avec de l'air, de l'oxygène ou du chlore et/ou avec de l'HF.

Par exemple, le catalyseur est de préférence soumis à une activation avec de l'air ou de l'oxygène et du HF à une température de 100 à 500°C, de préférence de 250 à 500°C et plus particulièrement de 300 à 400°C. La durée d'activation est de préférence de 1 à 200 h et plus particulièrement de 1 à 50 h.

Cette activation peut être suivie d'une étape d'activation de fluoration finale en présence d'un agent d'oxydation, d'HF et de composés organiques.

Le rapport molaire HF / composés organiques est de préférence de 2 à 40 et le rapport molaire agent d'oxydation / composés organiques est de préférence de 0,04 à 25. La température de l'activation finale est de préférence de 300 à 400°C et sa durée de préférence de 6 à 100 h.

La réaction de fluoration en phase gazeuse peut être effectuée :

- avec un rapport molaire HF / composé de formule (I) et/ou (II) de 3:1 à 150:1, de préférence de 4:1 à 125:1 et de manière plus particulièrement préférée de 5:1 à 100:1 ;
- avec un temps de contact de 3 à 100 s, de préférence 4 à 75 s et plus particulièrement 5 à 50 s (volume de catalyseur divisé par le flux entrant total, ajusté à la température et à la pression de fonctionnement) ;
- à une pression allant de la pression atmosphérique à 20 bar, de préférence de 2 à 18 bar et plus particulièrement de 3 à 15 bars;
- à une température (température du lit de catalyseur) de 200 à 450°C, de préférence de 250 à 400°C, et plus particulièrement de 280 à 380°C.

La durée de l'étape de réaction est typiquement de 10 à 8000 heures, de préférence de 50 à 5000 heures et de manière plus particulièrement préférée de 70 à 1000 heures.

Un agent oxydant, de préférence l'oxygène, peut éventuellement être ajouté lors de la réaction de fluoration. Le rapport molaire oxygène / composés organiques peut être de 0,005 à 2, de préférence de 0,01 à 1,5. L'oxygène peut être introduit pur ou sous forme d'air ou de mélange oxygène / azote. On peut également remplacer l'oxygène par du chlore.

## Méthode de sélection de l'agent d'extraction organique

La sélection de l'agent d'extraction organique est déterminée par l'utilisation du modèle Cosmo-RS implémenté dans le logiciel COSMOTHERM. Pour ce couple binaire sélectionné, un facteur de séparation est calculé pour chacun des solvants étudiés par l'équation suivante :

$$S_{1,2} = (\gamma_{1,S} * P1)/(\gamma_{2,S} * P2)$$

dans laquelle

$\gamma_{1,S}$ représente le coefficient d'activité du premier composé 1 dans l'agent d'extraction organique considéré à dilution infinie,

P1 représente la pression de vapeur saturante du premier composé 1,

$\gamma_{2,S}$ représente le coefficient d'activité du second composé 2 du couple binaire dans l'agent d'extraction organique considéré à dilution infinie,

P2 représente la pression de vapeur saturante du second composé.

Une capacité d'absorption est également calculée pour chacun des solvants étudiés et pour un couple binaire (1,2) considéré. La capacité d'absorption est calculée par la formule $C_{2,S} = 1/(\gamma_{2,S})$ dans laquelle $\gamma_{2,S}$ représente le coefficient d'activité du second composé du couple binaire considéré dans ledit agent d'extraction organique étudié à dilution infinie.

Les calculs sont répétés pour chaque agent d'extraction organique étudié. Des valeurs minimales de facteur de séparation et de capacité d'absorption sont identifiées afin de permettre une séparation suffisante entre le premier composé et le second composé du couple binaire (1,2) considéré. La pression de vapeur saturante est considérée pour une température de 25°C.

## Exemples

Pour purifier le 2,3,3,3-tetrafluoro-1-propene, le couple binaire 2,3,3,3-tetrafluoro-1-propene / trans-1,3,3,3-tetrafluoro-1-propene (1234ze-E) est considéré pour sélectionner l'agent d'extraction organique (voir tableau 1). Le mélange à purifier comprend 2,3,3,3-tetrafluoro-1-propene, 1,1,1,2,2-pentafluoropropane, et trans-1,3,3,3-tetrafluoro-1-propene (1234ze-E).

Tableau 1 - Capacité et Facteur de séparation de l'agent d'extraction organique

| Agent extraction organique | Capacité d'absorption (1234zeE) | Facteur de séparation (1234yf/1234zeE) | Facteur de séparation (245cb/1234zeE) |
|---|---|---|---|
| Ethylamine | 1,95 | 2,92 | 3,60 |
| Isopropylamine | 1,85 | 2,56 | 3,14 |
| Diethylether | 1,48 | 1,79 | 1,68 |
| n-propylamine | 1,86 | 2,61 | 3,14 |
| Diéthylamine | 1,57 | 1,88 | 2,18 |
| Diéthoxyméthane | 1,41 | 1,86 | 1,63 |
| Isopropylacétate | 1,42 | 2,15 | 1,79 |
| 3-pentylamine | 1,63 | 2,05 | 2,39 |
| 2-methoxyethanamine | 1,98 | 3,19 | 3,80 |
| tert-butylacetate | 1,37 | 2,10 | 1,63 |
| dioxane | 1,09 | 2,24 | 1,92 |
| 1,1-diethoxyethane | 1,54 | 1,90 | 1,78 |
| trimethoxymethane | 1,28 | 2,11 | 1,81 |
| n-pentylamine | 1,64 | 2,31 | 2,71 |
| 1,3-dioxane | 1,09 | 2,23 | 1,93 |
| sec-butylacetate | 1,45 | 2,12 | 1,79 |
| 1,2-diaminoethane | 1,29 | 4,43 | 6,88 |
| 1-methoxy-2-propanol | 0,87 | 2,31 | 2,15 |
| 1,2-propanediamine | 1,50 | 3,54 | 4,92 |

(suite)

| Agent extraction organique | Capacité d'absorption (1234zeE) | Facteur de séparation (1234yf/1234zeE) | Facteur de séparation (245cb/1234zeE) |
|---|---|---|---|
| n-butylacetate | 1,35 | 2,14 | 1,86 |
| 2-methoxy-1-propanol | 0,87 | 2,31 | 2,13 |
| hexanal | 1,09 | 1,98 | 1,72 |

## Revendications

1. Procédé de purification du 2,3,3,3-tétrafluoro-1-propène (1234yf) à partir d'une première composition comprenant du 2,3,3,3-tétrafluoro-1-propène, 1,1,1,2,2-pentafluoropropane (245cb) et trans-1,3,3,3-tetrafluoro-1-propene (1234ze-E), ledit procédé comprenant les étapes de:

   a) mise en contact de ladite première composition avec au moins un agent d'extraction organique pour former une seconde composition ;
   b) distillation extractive de ladite seconde composition pour former :

      i) une troisième composition comprenant ledit agent d'extraction organique et trans-1,3,3,3-tetrafluoro-1-propene (1234ze-E); et
      ii) un courant comprenant le 2,3,3,3-tétrafluoro-1-propène (1234yf) et le 1,1,1,2,2-pentafluoropropane (245cb),

   c) récupération et séparation de ladite troisième composition pour former un courant comprenant ledit agent d'extraction organique et un courant comprenant le trans-1,3,3,3-tetrafluoro-1-propene (1234ze-E) ; de préférence le courant comprenant ledit agent d'extraction organique est recyclé à l'étape a).

2. Procédé selon la revendication précédente **caractérisé en ce qu'**il comprend également une étape de récupération dudit courant ii) comprenant le 2,3,3,3-tétrafluoro-1-propène et le 1,1,1,2,2-pentafluoropropane (245cb) obtenu à l'étape b), et de distillation de celui-ci pour former un courant A comprenant 2,3,3,3-tétrafluoro-1-propène et un courant B comprenant 1,1,1,2,2-pentafluoropropane (245cb).

3. Procédé selon l'une quelconque des revendications précédentes **caractérisé en ce que** ladite première composition comprend également au moins un des composés sélectionnés parmi le groupe consistant en chlorométhane (40), 1,1-difluoroéthane (152a), 1,1,1,2-tetrafluoroethane (134a), chloropentafluoroéthane (115), trans-1,2,3,3,3-pentafluoropropene (1225ye-E), cis-1,2,3,3,3-pentafluoropropene (1225ye-Z), 3,3,3-trifluoropropene (1243zf).

4. Procédé selon la revendication précédente **caractérisé en ce que** lesdits au moins un des composés sélectionnés parmi le groupe consistant en chlorométhane (40), 1,1-difluoroéthane (152a), 1,1,1,2-tetrafluoroethane (134a), chloropentafluoroéthane (115), trans-1,2,3,3,3-pentafluoropropene (1225ye-E), cis-1,2,3,3,3-pentafluoropropene (1225ye-Z), 3,3,3-trifluoropropene (1243zf) sont également contenus dans lesdites seconde et troisième compositions ; l'étape c) du présent procédé étant la récupération et la séparation de ladite troisième composition pour former d'une part un courant comprenant ledit agent d'extraction organique et d'autre part un courant comprenant le trans-1,3,3,3-tetrafluoro-1-propene (1234ze-E) et ledit au moins un des composés sélectionnés parmi le groupe consistant en chlorométhane (40), 1,1-difluoroéthane (152a), 1,1,1,2-tetrafluoroethane (134a), chloropentafluoroéthane (115), trans-1,2,3,3,3-pentafluoropropene (1225ye-E), cis-1,2,3,3,3-pentafluoropropene (1225ye-Z), 3,3,3-trifluoropropene (1243zf) ; de préférence le courant comprenant ledit agent d'extraction organique est recyclé à l'étape a).

5. Procédé selon l'une quelconque des revendications précédentes **caractérisé en ce que** ledit agent d'extraction organique est un solvant choisi parmi le groupe consistant en hydrohalocarbure, alcool, cétone, amine, ester, éther, aldéhyde, nitrile, carbonate, thioalkyle, amide, hétérocycle; ou l'agent d'extraction organique est triethylfluorosilane ; de préférence, l'agent d'extraction organique est choisi parmi le groupe consistant en amine, éther, ester, aldéhyde, cétone, alcool et hétérocycle.

**6.** Procédé selon l'une quelconque des revendications précédentes **caractérisé en ce que** ledit agent d'extraction organique a un facteur de séparation $S_{1,2}$ supérieur ou égal à 1,1, ledit facteur de séparation étant calculé par la formule $S_{1,2} = (\gamma_{1,S}*P1)/(\gamma_{2,S}*P2)$ dans laquelle

$\gamma_{1,S}$ représente le coefficient d'activité du 2,3,3,3-tétrafluoro-1-propène dans ledit agent d'extraction organique à dilution infinie,
P1 représente la pression de vapeur saturante du 2,3,3,3-tétrafluoro-1-propène,
$\gamma_{2,S}$ représente le coefficient d'activité du trans-1,3,3,3-tetrafluoro-1-propene (1234ze-E) dans ledit agent d'extraction organique à dilution infinie,
P2 représente la pression de vapeur saturante du trans-1,3,3,3-tetrafluoro-1-propene (1234ze-E) ;
avantageusement, le facteur de séparation est supérieur ou égal à 1,2, de préférence supérieur ou égal à 1,4, plus préférentiellement supérieur ou égal à 1,6, en particulier supérieur ou égal à 1,8, plus particulièrement supérieur ou égal à 2,0.

**7.** Procédé selon l'une quelconque des revendications précédentes **caractérisé en ce que** ledit agent d'extraction organique a un facteur de séparation $S_{1,2}$ supérieur ou égal à 1,1, ledit facteur de séparation étant calculé par la formule $S_{1,2} = (\gamma_{1,S}*P1)/(\gamma_{2,S}*P2)$ dans laquelle $\gamma_{1,S}$ représente le coefficient d'activité du 1,1,1,2,2-pentafluoropropane (245cb) dans ledit agent d'extraction organique à dilution infinie,

P1 représente la pression de vapeur saturante du 1,1,1,2,2-pentafluoropropane (245cb), $\gamma_{2,S}$ représente le coefficient d'activité du trans-1,3,3,3-tetrafluoro-1-propene (1234ze-E) dans ledit agent d'extraction organique à dilution infinie,
P2 représente la pression de vapeur saturante du trans-1,3,3,3-tetrafluoro-1-propene (1234ze-E) ; avantageusement, le facteur de séparation est supérieur ou égal à 1,2, de préférence supérieur ou égal à 1,4, plus préférentiellement supérieur ou égal à 1,6, en particulier supérieur ou égal à 1,8, plus particulièrement supérieur ou égal à 2,0.

**8.** Procédé selon l'une quelconque des revendications précédentes **caractérisé en ce que** ledit agent d'extraction organique a une capacité d'absorption $C_{2,S}$ supérieure ou égale à 0,20, ladite capacité d'absorption étant calculé par la formule $C_{2,S} = 1/(\gamma_{2,S})$ dans laquelle $\gamma_{2,S}$ représente le coefficient d'activité dudit au moins un des composés consistant en trans-1,3,3,3-tetrafluoro-1-propene (1234ze-E) dans ledit agent d'extraction organique à dilution infinie, de préférence $\gamma_{2,S}$ représente le coefficient d'activité dudit au moins un des composés consistant en trans-1,3,3,3-tetrafluoro-1-propene (1234ze-E), dans ledit agent d'extraction organique à dilution infinie ;
avantageusement, la capacité d'absorption $C_{2,S}$ est supérieure ou égale à 0,40, de préférence supérieure ou égale à 0,60, plus préférentiellement supérieure ou égale à 0,80, en particulier supérieure ou égale à 1,0.

**9.** Procédé selon l'une quelconque des revendications précédentes **caractérisé en ce que** ledit agent d'extraction organique est choisi parmi le groupe consistant en éthylamine, isopropylamine, diethylether, n-propylamine, diethylamine, propanone, methylacetate, butanone, diethoxymethane, isopropylacetate, 3-pentylamine, 2-methoxyethanamine, tert-butylacetate, dioxane, 1,1-diethoxyethane, trimethoxymethane, n-pentylamine, 1,3-dioxane, sec-butylacetate, 1,2-diaminoethane, 1-methoxy-2-propanol, 1,2-propanediamine, n-butylacetate, 2-methoxy-1-propanol, hexanal. De préférence, ledit agent d'extraction organique est choisi parmi le groupe consistant en éthylamine, isopropylamine, diethylether, n-propylamine, diethylamine, diethoxymethane, isopropylacetate, 3-pentylamine, 2-methoxyethanamine, tert-butylacetate, dioxane, 1,1-diethoxyethane, trimethoxymethane, n-pentylamine, 1,3-dioxane, sec-butylacetate, 1,2-diaminoethane, 1-methoxy-2-propanol, 1,2-propanediamine, n-butylacetate, 2-methoxy-1-propanol, hexanal.

**10.** Procédé selon l'une quelconque des revendications précédentes **caractérisé en ce que** le courant ii) comprenant le 2,3,3,3-tetrafluoro-1-propène et 1,1,1,2,2-pentafluoropropane (245cb) formé à l'étape b) est récupéré et est dépourvu de trans-1,3,3,3-tetrafluoro-1-propene (1234ze-E).

**11.** Procédé selon l'une quelconque des revendications précédentes **caractérisé en ce qu'**il comprend préalablement à l'étape a) les étapes :

i') mise en oeuvre d'une composition comprenant 2,3,3,3-tetrafluoro-1-propène, des impuretés ayant un point d'ébullition inférieur au point d'ébullition du 2,3,3,3-tetrafluoro-1-propène, 1,1,1,2,2-pentafluoropropane (245cb), et trans-1,3,3,3-tetrafluoro-1-propene (1234ze-E), optionnellement ou non au moins un des composés sélectionnés parmi le groupe consistant en chlorométhane (40), 1,1-difluoroéthane (152a), 1,1,1,2-tetrafluoroethane (134a), chloropentafluoroéthane (115), trans-1,2,3,3,3-pentafluoropropene (1225ye-E), cis-1,2,3,3,3-pentafluoropropene (1225ye-Z), 3,3,3-trifluoropropene (1243zf), et optionnellement ou non des impuretés lourdes ;

ii') distillation de ladite composition de l'étape i) pour éliminer en tête de colonne des impuretés ayant un point d'ébullition inférieur au point d'ébullition du 2,3,3,3-tetrafluoro-1-propène et former un premier courant comprenant 2,3,3,3-tetrafluoro-1-propène, 1,1,1,2,2-pentafluoropropane (245cb), et trans-1,3,3,3-tetrafluoro-1-propène (1234ze-E), optionnellement ou non au moins un des composés sélectionnés parmi le groupe consistant en chlorométhane (40), 1,1-difluoroéthane (152a), 1,1,1,2-tetrafluoroethane (134a), chloropentafluoroéthane (115), trans-1,2,3,3,3-pentafluoropropene (1225ye-E), cis-1,2,3,3,3-pentafluoropropene (1225ye-Z), 3,3,3-trifluoropropene (1243zf), et optionnellement ou non des impuretés lourdes, récupéré en bas de colonne de distillation ;

iii') optionnellement ou non, distillation dudit premier courant récupéré en bas de colonne de distillation à l'étape ii') pour récupérer en tête de colonne un second courant comprenant du 2,3,3,3-tetrafluoro-1-propène, 1,1,1,2,2-pentafluoropropane (245cb), et trans-1,3,3,3-tetrafluoro-1-propene (1234ze-E), optionnellement ou non au moins un des composés sélectionnés parmi le groupe consistant en chlorométhane (40), 1,1-difluoroéthane (152a), 1,1,1,2-tetrafluoroethane (134a), chloropentafluoroéthane (115), trans-1,2,3,3,3-pentafluoropropene (1225ye-E), cis-1,2,3,3,3-pentafluoropropene (1225ye-Z), 3,3,3-trifluoropropene (1243zf), et en bas de colonne de distillation un courant comprenant les impuretés lourdes ;

ledit premier courant récupéré à l'étape ii') ou ledit second courant récupéré à l'étape iii') correspondent à ladite première composition mise en oeuvre à l'étape a).

12. Procédé de production et de purification du 2,3,3,3-tetrafluoro-1-propène comprenant les étapes de :

A) fluoration en présence d'un catalyseur d'un composé de formule (I) $CX(Y)_2-CX(Y)_m-CH_mXY$ dans laquelle X et Y représentent indépendamment un atome d'hydrogène, de fluor ou de chlore et m = 0 ou 1 ; et/ou fluoration catalytique en présence d'un catalyseur d'un composé de formule $(CX_nY_{3-n})CH_pX_{1-p}CH_mX_{2-m}$ (II) dans lequel X est indépendamment les uns des autres Cl, F, I ou Br ; Y est indépendamment les uns des autres H, Cl, F, I ou Br ; n est 1, 2 ou 3 ; et m est 0, 1 ou 2 ; et p est 0 ou 1 ;

B) récupération d'un courant comprenant du 2,3,3,3-tetrafluoro-1-propène, 1,1,1,2,2-pentafluoropropane (245cb), trans-1,3,3,3-tetrafluoro-1-propene (1234ze-E), et optionnellement ou non au moins un des composés sélectionnés parmi le groupe consistant en chlorométhane (40), 1,1-difluoroéthane (152a), 1,1,1,2-tetrafluoroethane (134a), chloropentafluoroéthane (115), trans-1,2,3,3,3-pentafluoropropene (1225ye-E), cis-1,2,3,3,3-pentafluoropropene (1225ye-Z), 3,3,3-trifluoropropene (1243zf) ;

C) mise en oeuvre du procédé de purification du 2,3,3,3-tetrafluoro-1-propène selon l'une quelconque des revendications 1 à 11 à partir du courant récupéré à l'étape B).

## Patentansprüche

1. Verfahren zur Reinigung von 2,3,3,3-Tetrafluor-1-propen (1234yf) ausgehend von einer ersten Zusammensetzung, die 2,3,3,3-Tetrafluor-1-propen, 1,1,1,2,2-Pentafluorpropan (245cb) und trans-1,3,3,3-Tetrafluor-1-propen (1234ze-E) umfasst, wobei das Verfahren die folgenden Schritte umfasst:

a) Inkontaktbringen der ersten Zusammensetzung mit mindestens einem organischen Extraktionsmittel unter Herstellung einer zweiten Zusammensetzung;
b) extraktive Destillation der zweiten Zusammensetzung unter Herstellung:

i. einer dritten Zusammensetzung, die das organische Extraktionsmittel und trans-1,3,3,3-Tetrafluor-1-propen (1234ze-E) umfasst, und
ii. eines Stroms, der 2,3,3,3-Tetrafluor-1-propen (1234yf) und 1,1,1,2,2-Pentafluorpropan (245cb) umfasst;

c) Gewinnung und Trennung der dritten Zusammensetzung unter Herstellung eines Stroms, der das organische Extraktionsmittel umfasst, und eines Stroms, der trans-1,3,3,3-Tetrafluor-1-propen (1234ze-E) umfasst, wobei vorzugsweise der das organische Extraktionsmittel umfassende Strom zum Schritt a) rückgeführt wird.

2. Verfahren nach dem vorhergehenden Anspruch, **dadurch gekennzeichnet, dass** es außerdem einen Schritt der Gewinnung des in Schritt b) erhaltenen Stroms ii), der 2,3,3,3-Tetrafluor-1-propen und 1,1,1,2,2-Pentafluorpropan (245cb) umfasst, und der Destillation desselben unter Herstellung eines 2,3,3,3-Tetrafluor-1-propen umfassenden Stroms **A** und eines 1,1,1,2,2-Pentafluorpropan (245cb) umfassenden Stroms **B** umfasst.

3. Verfahren nach einem der vorhergehenden Ansprüche, **dadurch gekennzeichnet, dass** die erste Zusammensetzung außerdem mindestens eine der aus der aus Chlormethan (40), 1,1-Difluorethan (152a), 1,1,1,2-Tetrafluorethan (134a), Chlorpentafluorethan (115), trans-1,2,3,3,3-Pentafluorpropen (1225ye-E), cis-1,2,3,3,3-Pentafluorpropen (1225ye-Z), 3,3,3-Trifluorpropen (1243zf) bestehenden Gruppe ausgewählten Verbindungen umfasst.

4. Verfahren nach dem vorhergehenden Anspruch, **dadurch gekennzeichnet, dass** die mindestens eine der aus der aus Chlormethan (40), 1,1-Difluorethan (152a), 1,1,1,2-Tetrafluorethan (134a), Chlorpentafluorethan (115), trans-1,2,3,3,3-Pentafluorpropen (1225ye-E), cis-1,2,3,3,3-Pentafluorpropen (1225ye-Z), 3,3,3-Trifluorpropen (1243zf) bestehenden Gruppe ausgewählten Verbindungen auch in der zweiten und der dritten Zusammensetzung enthalten sind; und es sich bei Schritt c) des vorliegenden Verfahrens um die Gewinnung und Trennung der dritten Zusammensetzung unter Herstellung einerseits eines Stroms, der das organische Extraktionsmittel umfasst, und andererseits eines Stroms, der trans-1,3,3,3-Tetrafluor-1-propen (1234ze-E) und die mindestens eine der aus der aus Chlormethan (40), 1,1-Difluorethan (152a), 1,1,1,2-Tetrafluorethan (134a), Chlorpentafluorethan (115), trans-1,2,3,3,3-Pentafluorpropen (1225ye-E), cis-1,2,3,3,3-Pentafluorpropen (1225ye-Z), 3,3,3-Trifluorpropen (1243zf) bestehenden Gruppe ausgewählten Verbindungen umfasst, handelt, wobei vorzugsweise der Strom, der das organische Extraktionsmittel umfasst, zum Schritt a) rückgeführt wird.

5. Verfahren nach einem der vorhergehenden Ansprüche, **dadurch gekennzeichnet, dass** das organische Extraktionsmittel ein Lösungsmittel ist, das aus der aus halogeniertem Kohlenwasserstoff, Alkohol, Keton, Amin, Ester, Ether, Aldehyd, Nitril, Carbonat, Thioalkyl, Amid, Heterozyklus bestehenden Gruppe ausgewählt ist; oder das organische Extraktionsmittel Triethylfluorsilan ist, wobei vorzugsweise das organische Extraktionsmittel aus der aus Amin, Ether, Ester, Aldehyd, Keton, Alkohol und Heterozyklus bestehenden Gruppe ausgewählt ist.

6. Verfahren nach einem der vorhergehenden Ansprüche, **dadurch gekennzeichnet, dass** das organische Extraktionsmittel einen Trennfaktor $S_{1,2}$ von größer als oder gleich 1,1 aufweist, wobei der Trennfaktor durch die Formel $S_{1,2} = (\gamma_{1,S}*P1)/(\gamma_{2,s}*P2)$ berechnet wird, worin $\gamma_{1,S}$ den Aktivitätskoeffizienten von 2,3,3,3-Tetrafluor-1-propen in dem organischen Extraktionsmittel bei unendlicher Verdünnung darstellt,

P1 den Sättigungsdampfdruck von 2,3,3,3-Tetrafluor-1-propen darstellt,
$\gamma_{2,S}$ den Aktivitätskoeffizienten von trans-1,3,3,3-Tetrafluor-1-propen (1234ze-E) in dem organischen Extraktionsmittel bei unendlicher Verdünnung darstellt, P2 den Sättigungsdampfdruck von trans-1,3,3,3-Tetrafluor-1-propen (1234ze-E) darstellt,
wobei vorteilhafterweise der Trennfaktor größer als oder gleich 1,2, vorzugsweise größer als oder gleich 1,4, stärker bevorzugt größer als oder gleich 1,6, insbesondere größer als oder gleich 1,8, ganz besonders größer als oder gleich 2,0 ist.

7. Verfahren nach einem der vorhergehenden Ansprüche, **dadurch gekennzeichnet, dass** das organische Extraktionsmittel einen Trennfaktor $S_{1,2}$ von größer als oder gleich 1,1 aufweist, wobei der Trennfaktor durch die Formel $S_{1,2} = (\gamma_{1,S}*P1)/(\gamma_{2,s}*P2)$ berechnet wird, worin $\gamma_{1,S}$ den Aktivitätskoeffizienten von 1,1,1,2,2-Pentafluorpropan (245cb) in dem organischen Extraktionsmittel bei unendlicher Verdünnung darstellt, P1 den Sättigungsdampfdruck von 1,1,1,2,2-Pentafluorpropan (245cb) darstellt,
$\gamma_{2,S}$ den Aktivitätskoeffizienten von trans-1,3,3,3-Tetrafluor-1-propen (1234ze-E) in dem organischen Extraktionsmittel bei unendlicher Verdünnung darstellt, P2 den Sättigungsdampfdruck von trans-1,3,3,3-Tetrafluor-1-propen (1234ze-E) darstellt, wobei vorteilhafterweise der Trennfaktor größer als oder gleich 1,2, vorzugsweise größer als oder gleich 1,4, stärker bevorzugt größer als oder gleich 1,6, insbesondere größer als oder gleich 1,8, ganz besonders größer als oder gleich 2,0 ist.

8. Verfahren nach einem der vorhergehenden Ansprüche, **dadurch gekennzeichnet, dass** das organische Extraktionsmittel eine Absorptionskapazität $C_{2,S}$ von größer als oder gleich 0,20 aufweist, wobei die Absorptionskapazität durch die Formel $C_{2,S} = 1/(\gamma_{2,S})$ berechnet wird, worin $\gamma_{2,S}$ den Aktivitätskoeffizienten der mindestens einen der aus trans-1,3,3,3-Tetrafluor-1-propen (1234ze-E) bestehenden Verbindungen in dem organischen Extraktionsmittel bei unendlicher Verdünnung darstellt, wobei vorzugsweise $\gamma_{2,S}$ den Aktivitätskoeffizienten der mindestens einen der aus trans-1,3,3,3-Tetrafluor-1-propen (1234ze-E) bestehenden Verbindungen in dem organischen Extraktionsmittel bei unendlicher Verdünnung darstellt;
wobei vorteilhafterweise die Absorptionskapazität $C_{2,S}$ größer als oder gleich 0,40, vorzugsweise größer als oder gleich 0,60, stärker bevorzugt größer als oder gleich 0,80, insbesondere größer als oder gleich 1,0 ist.

9. Verfahren nach einem der vorhergehenden Ansprüche, **dadurch gekennzeichnet, dass** das organische Extraktionsmittel aus der aus Ethylamin, Isopropylamin, Diethylether, n-Propylamin, Diethylamin, Propanon, Methylacetat, Butanon, Diethoxymethan, Isopropylacetat, 3-Pentylamin, 2-Methoxyethanamin, tert-Butylacetat, Dioxan, 1,1-Diethoxyethan, Trimethoxymethan, n-Pentylamin, 1,3-Dioxan, sek-Butylacetat, 1,2-Diaminoethan, 1-Methoxy-2-propanol, 1,2-Propandiamin, n-Butylacetat, 2-Methoxy-1-propanol, Hexanal bestehenden Gruppe ausgewählt ist; vorzugsweise ist das organische Extraktionsmittel aus der aus Ethylamin, Isopropylamin, Diethylether, n-Propylamin, Diethylamin, Diethoxymethan, Isopropylacetat, 3-Pentylamin, 2-Methoxyethanamin, tert-Butylacetat, Dioxan, 1,1-Diethoxyethan, Trimethoxymethan, n-Pentylamin, 1,3-Dioxan, sek-Butylacetat, 1,2-Diaminoethan, 1-Methoxy-2-propanol, 1,2-Propandiamin, n-Butylacetat, 2-Methoxy-1-propanol, Hexanal bestehenden Gruppe ausgewählt.

10. Verfahren nach einem der vorhergehenden Ansprüche, **dadurch gekennzeichnet, dass** der in Schritt b) hergestellte Strom ii), der 2,3,3,3-Tetrafluor-1-propen und 1,1,1,2,2-Pentafluorpropan (245cb) umfasst, gewonnen wird und frei von trans-1,3,3,3-Tetrafluor-1-propen (1234ze-E) ist.

11. Verfahren nach einem der vorhergehenden Ansprüche, **dadurch gekennzeichnet, dass** es vor Schritt a) die folgenden Schritte umfasst:

i') Einsetzen einer Zusammensetzung, die 2,3,3,3-Tetrafluor-1-propen, Verunreinigungen mit einem Siedepunkt unterhalb des Siedepunkts von 2,3,3,3-Tetrafluor-1-propen, 1,1,1,2,2-Pentafluorpropan (245cb) und trans-1,3,3,3-Tetrafluor-1-propen (1234ze-E), gegebenenfalls mindestens eine der aus der aus Chlormethan (40), 1,1-Difluorethan (152a), 1,1,1,2-Tetrafluorethan (134a), Chlorpentafluorethan (115), trans-1,2,3,3,3-Pentafluorpropen (1225ye-E), cis-1,2,3,3,3-Pentafluorpropen (1225ye-Z), 3,3,3-Trifluorpropen (1243zf) bestehenden Gruppe ausgewählten Verbindungen und gegebenenfalls schwere Verunreinigungen umfasst;

ii') Destillation der Zusammensetzung von Schritt i), um am Kopf der Kolonne die Verunreinigungen mit einem Siedepunkt unterhalb des Siedepunkts von 2,3,3,3-Tetrafluor-1-propen zu entfernen und einen ersten Strom herzustellen, der 2,3,3,3-Tetrafluor-1-propen, 1,1,1,2,2-Pentafluorpropan (245cb) und trans-1,3,3,3-Tetrafluor-1-propen (1234ze-E), gegebenenfalls mindestens eine der aus der aus Chlormethan (40), 1,1-Difluorethan (152a), 1,1,1,2-Tetrafluorethan (134a), Chlorpentafluorethan (115), trans-1,2,3,3,3-Pentafluorpropen (1225ye-E), cis-1,2,3,3,3-Pentafluorpropen (1225ye-Z), 3,3,3-Trifluorpropen (1243zf) bestehenden Gruppe ausgewählten Verbindungen und gegebenenfalls schwere Verunreinigungen umfasst und am Boden der Destillationskolonne gewonnen wird;

iii') gegebenenfalls Destillation des im Schritt ii') am Boden der Destillationskolonne gewonnenen ersten Stroms, um am Kopf der Kolonne einen zweiten Strom, der 2,3,3,3-Tetrafluor-1-propen, 1,1,1,2,2-Pentafluorpropan (245cb) und trans-1,3,3,3-Tetrafluor-1-propen (1234ze-E), gegebenenfalls mindestens eine der aus der aus Chlormethan (40), 1,1-Difluorethan (152a), 1,1,1,2-Tetrafluorethan (134a), Chlorpentafluorethan (115), trans-1,2,3,3,3-Pentafluorpropen (1225ye-E), cis-1,2,3,3,3-Pentafluorpropen (1225ye-Z), 3,3,3-Trifluorpropen (1243zf) bestehenden Gruppe ausgewählten Verbindungen umfasst, und am Boden der Destillationskolonne einen Strom, der die schweren Verunreinigungen enthält, zu gewinnen;

wobei der im Schritt ii') gewonnene erste Strom oder der im Schritt iii') gewonnene zweite Strom der ersten Zusammensetzung entspricht, die im Schritt a) eingesetzt wird.

12. Verfahren zur Produktion und Reinigung von 2,3,3,3-Tetrafluor-1-propen, umfassend die Schritte:

A) Fluorierung in Gegenwart eines Katalysators einer Verbindung der Formel (I) $CX(Y)_2$-$CX(Y)_m$-$CH_mXY$, worin X und Y unabhängig ein Wasserstoff-, Fluor- oder Chloratom darstellen und m = 0 oder 1; und/oder katalytische Fluorierung in Gegenwart eines Katalysators für eine Verbindung der Formel $(CX_nY_{3-n})CH_pX_{1-p}CH_mX_{2-m}$ (II), worin X unabhängig voneinander Cl, F, I oder Br ist; Y unabhängig voneinander H, Cl, F, I oder Br ist; n 1, 2 oder 3 ist und m 0, 1 oder 2 ist und p 0 oder 1 ist;

B) Gewinnung eines Stroms, der 2,3,3,3-Tetrafluor-1-propen, 1,1,1,2,2-Pentafluorpropan (245cb), trans-1,3,3,3-Tetrafluor-1-propen (1234zeE) und gegebenenfalls mindestens eine der aus der aus Chlormethan (40), 1,1-Difluorethan (152a), 1,1,1,2-Tetrafluorethan (134a), Chlorpentafluorethan (115), trans-1,2,3,3,3-Pentafluorpropen (1225ye-E), cis-1,2,3,3,3-Pentafluorpropen (1225ye-Z), 3,3,3-Trifluorpropen (1243zf) bestehenden Gruppe ausgewählten Verbindungen umfasst;

C) Durchführen des Verfahrens zur Reinigung von 2,3,3,3-Tetrafluor-1-propen nach einem der Ansprüche 1 bis 11 ausgehend von dem im Schritt B) gewonnenen Strom.

**Claims**

1. Process for purifying 2,3,3,3-tetrafluoro-1-propene (1234yf) using a first composition comprising 2,3,3,3-tetrafluoro-1-propene, 1,1,1,2,2-pentafluoropropane (245cb) and trans-1,3,3,3-tetrafluoro-1-propene (1234ze-E), said process comprising the steps of:

   a) placing said first composition in contact with at least one organic extracting agent to form a second composition;
   b) extractive distillation of said second composition to form:

       i) a third composition comprising said organic extracting agent and trans-1,3,3,3-tetrafluoro-1-propene (1234ze-E); and
       ii) a stream comprising 2,3,3,3-tetrafluoro-1-propene (1234yf) and 1,1,1,2,2-pentafluoropropane (245cb);

   c) recovery and separation of said third composition to form a stream comprising said organic extracting agent and a stream comprising trans-1,3,3,3-tetrafluoro-1-propene (1234ze-E); preferably, the stream comprising said organic extracting agent is recycled into step a).

2. Process according to the preceding claim, **characterized in that** it also comprises a step of recovering said stream ii) comprising 2,3,3,3-tetrafluoro-1-propene and 1,1,1,2,2-pentafluoropropane (245cb) obtained in step b), and of distilling said stream to form a stream **A** comprising 2, 3, 3, 3-tetrafluoro-1-propene and a stream **B** comprising 1,1,1,2,2-pentafluoropropane (245cb).

3. Process according to either of the preceding claims, **characterized in that** said first composition also comprises at least one of the compounds chosen from the group consisting of chloromethane (40), 1,1-difluoroethane (152a), 1,1,1,2-tetrafluoroethane (134a), chloropentafluoroethane (115), trans-1,2,3,3,3-pentafluoropropene (1225ye-E), cis-1,2,3,3,3-pentafluoropropene (1225ye-Z) and 3,3,3-trifluoropropene (1243zf).

4. Process according to the preceding claim, **characterized in that** said at least one of the compounds chosen from the group consisting of chloromethane (40), 1,1-difluoroethane (152a), 1,1,1,2-tetrafluoroethane (134a), chloropentafluoroethane (115), trans-1,2,3,3,3-pentafluoropropene (1225ye-E), cis-1,2,3,3,3-pentafluoropropene (1225ye-Z) and 3,3,3-trifluoropropene (1243zf) are also contained in said second and third compositions; step c) of the present process being the recovery and separation of said third composition to form, on the one hand, a stream comprising said organic extracting agent and, on the other hand, a stream comprising trans-1,3,3,3-tetrafluoro-1-propene (1234ze-E) and said at least one of the compounds chosen from the group consisting of chloromethane (40), 1,1-difluoroethane (152a), 1,1,1,2-tetrafluoroethane (134a), chloropentafluoroethane (115), trans-1,2,3,3,3-pentafluoropropene (1225ye-E), cis-1,2,3,3,3-pentafluoropropene (1225ye-Z) and 3,3,3-trifluoropropene (1243zf); preferably, the stream comprising said organic extracting agent is recycled into step a).

5. Process according to either of the preceding claims, **characterized in that** said organic extracting agent is a solvent chosen from the group consisting of halohydrocarbon, alcohol, ketone, amine, ester, ether, aldehyde, nitrile, carbonate, thioalkyl, amide and heterocycle; or the organic extracting agent is triethylfluorosilane; preferably, the organic extracting agent is chosen from the group consisting of amine, ether, ester, aldehyde, ketone, alcohol and heterocycle.

6. Process according to any one of the preceding claims, **characterized in that** said organic extracting agent has a separation factor $S_{1,2}$ of greater than or equal to 1.1, said separation factor being calculated by the formula $S_{1,2} = (\gamma_{1,s}*P1)/(\gamma_{2,s}*P2)$ in which

   $\gamma_{1,s}$ represents the activity coefficient of 2,3,3,3-tetrafluoro-1-propene in said organic extracting agent at infinite dilution;
   P1 represents the saturating vapor pressure of 2,3,3,3-tetrafluoro-1-propene;
   $\gamma_{2,s}$ represents the activity coefficient of trans-1,3,3,3-tetrafluoro-1-propene (1234ze-E) in said organic extracting agent at infinite dilution;
   P2 represents the saturating vapor pressure of trans-1,3,3,3-tetrafluoro-1-propene (1234ze-E); advantageously, the separation factor is greater than or equal to 1.2, preferably greater than or equal to 1.4, more preferentially greater than or equal to 1.6, in particular greater than or equal to 1.8, more particularly greater than or equal to 2.0.

7. Process according to any one of the preceding claims, **characterized in that** said organic extracting agent has a separation factor $S_{1,2}$ of greater than or equal to 1.1, said separation factor being calculated by the formula $S_{1,2} = $

$(\gamma_{1,s} * P1)/(\gamma_{2,s} * P2)$ in which

$\gamma_{1,s}$ represents the activity coefficient of 1,1,1,2,2-pentafluoropropane (245cb) in said organic extracting agent at infinite dilution;

P1 represents the saturating vapor pressure of 1,1,1,2,2-pentafluoropropane (245cb);

$\gamma_{2,s}$ represents the activity coefficient of trans-1,3,3,3-tetrafluoro-1-propene (1234ze-E) in said organic extracting agent at infinite dilution;

P2 represents the saturating vapor pressure of trans-1,3,3,3-tetrafluoro-1-propene (1234ze-E); advantageously, the separation factor is greater than or equal to 1.2, preferably greater than or equal to 1.4, more preferentially greater than or equal to 1.6, in particular greater than or equal to 1.8, more particularly greater than or equal to 2.0.

8. Process according to any one of the preceding claims, **characterized in that** said organic extracting agent has an absorption capacity $C_{2,s}$ of greater than or equal to 0.20, said absorption capacity being calculated by the formula $C_{2,s} = 1/(\gamma_{2,s})$ in which $\gamma_{2,s}$ represents the activity coefficient of said at least one of the compounds consisting of trans-1,3,3,3-tetrafluoro-1-propene (1234ze-E) in said organic extracting agent at infinite dilution; preferably, $\gamma_{2,s}$ represents the activity coefficient of said at least one of the compounds consisting of trans-1,3,3,3-tetrafluoro-1-propene (1234ze-E), in said organic extracting agent at infinite dilution;

advantageously, the absorption capacity $C_{2,s}$ is greater than or equal to 0.40, preferably greater than or equal to 0.60, more preferentially greater than or equal to 0.80, in particular greater than or equal to 1.0.

9. Process according to any one of the preceding claims, **characterized in that** said organic extracting agent is chosen from the group consisting of ethylamine, isopropylamine, diethyl ether, n-propylamine, diethylamine, propanone, methyl acetate, butanone, diethoxymethane, isopropyl acetate, 3-pentylamine, 2-methoxyethanamine, tert-butyl acetate, dioxane, 1,1-diethoxyethane, trimethoxymethane, n-pentylamine, 1,3-dioxane, sec-butyl acetate, 1,2-diaminoethane, 1-methoxy-2-propanol, 1,2-propanediamine, n-butyl acetate, 2-methoxy-1-propanol and hexanal; preferably, said organic extracting agent is chosen from the group consisting of ethylamine, isopropylamine, diethyl ether, n-propylamine, diethylamine, diethoxymethane, isopropyl acetate, 3-pentylamine, 2-methoxyethanamine, tert-butyl acetate, dioxane, 1,1-diethoxyethane, trimethoxymethane, n-pentylamine, 1,3-dioxane, sec-butyl acetate, 1,2-diaminoethane, 1-methoxy-2-propanol, 1,2-propanediamine, n-butyl acetate, 2-methoxy-1-propanol and hexanal.

10. Process according to any one of the preceding claims, **characterized in that** the stream ii) comprising 2,3,3,3-tetrafluoro-1-propene and 1,1,1,2,2-pentafluoropropane (245cb) formed in step b) is recovered and is free of trans-1,3,3,3-tetrafluoro-1-propene (1234ze-E) .

11. Process according to any one of the preceding claims, **characterized in that** it comprises, prior to step a), the following steps:

i') use of a composition comprising 2,3,3,3-tetrafluoro-1-propene, impurities with a boiling point below the boiling point of 2,3,3,3-tetrafluoro-1-propene, 1,1,1,2,2-pentafluoropropane (245cb), and trans-1,3,3,3-tetrafluoro-1-propene (1234ze-E), optionally or not at least one of the compounds chosen from the group consisting of chloromethane (40), 1,1-difluoroethane (152a), 1,1,1,2-tetrafluoroethane (134a), chloropentafluoroethane (115), trans-1,2,3,3,3-pentafluoropropene (1225ye-E), cis-1,2,3,3,3-pentafluoropropene (1225ye-Z), 3,3,3-trifluoropropene (1243zf), and optionally or not heavy impurities;

ii') distillation of said composition from step i) to remove, at the top of the column, impurities with a boiling point below the boiling point of 2,3,3,3-tetrafluoro-1-propene and to form a first stream comprising 2,3,3,3-tetrafluoro-1-propene, 1,1,1,2,2-pentafluoropropane (245cb) and trans-1,3,3,3-tetrafluoro-1-propene (1234ze-E), optionally or not at least one of the compounds chosen from the group consisting of chloromethane (40), 1,1-difluoroethane (152a), 1,1,1,2-tetrafluoroethane (134a), chloropentafluoroethane (115), trans-1,2,3,3,3-pentafluoropropene (1225ye-E), cis-1,2,3,3,3-pentafluoropropene (1225ye-Z), 3,3,3-trifluoropropene (1243zf), and optionally or not heavy impurities, recovered at the bottom of the distillation column;

iii') optionally or not, distillation of said first stream recovered at the bottom of the distillation column in step ii') to recover, at the top of the column, a second stream comprising 2,3,3,3-tetrafluoro-1-propene, 1,1,1,2,2-pentafluoropropane (245cb) and trans-1,3,3,3-tetrafluoro-1-propene (1234ze-E), optionally or not at least one of the compounds chosen from the group consisting of chloromethane (40), 1,1-difluoroethane (152a), 1,1,1,2-tetrafluoroethane (134a), chloropentafluoroethane (115), trans-1,2,3,3,3-pentafluoropropene (1225ye-E), cis-1,2,3,3,3-pentafluoropropene (1225ye-Z) and 3,3,3-trifluoropropene (1243zf), and, at the bottom of the distillation column, a stream comprising the heavy impurities;

said first stream recovered in step ii') or said second stream recovered in step iii') corresponding to said first composition used in step a).

12. Process for producing and purifying 2,3,3,3-tetrafluoro-1-propene, comprising the steps of:

A) fluorination in the presence of a catalyst for a compound of formula (I) $CX(Y)_2$-$CX(Y)_m$-$CH_mXY$ in which X and Y independently represent a hydrogen, fluorine or chlorine atom and m = 0 or 1; and/or catalytic fluorination in the presence of a catalyst for a compound of formula $(CX_nY_{3-n})CH_pX_{1-p}CH_mX_{2-m}$ (II) in which X is, independently of each other, Cl, F, I or Br; Y is, independently of each other, H, Cl, F, I or Br; n is 1, 2 or 3; and m is 0, 1 or 2; and p is 0 or 1;

B) recovery of a stream comprising 2,3,3,3-tetrafluoro-1-propene, 1,1,1,2,2-pentafluoropropane (245cb), trans-1,3,3,3-tetrafluoro-1-propene (1234ze-E) and optionally or not at least one of the compounds chosen from the group consisting of chloromethane (40), 1,1-difluoroethane (152a), 1,1,1,2-tetrafluoroethane (134a), chloropentafluoroethane (115), trans-1,2,3,3,3-pentafluoropropene (1225ye-E), cis-1,2,3,3,3-pentafluoropropene (1225ye-Z) and 3,3,3-trifluoropropene (1243zf);

C) implementation of the process for purifying 2,3,3,3-tetrafluoro-1-propene according to any one of Claims 1 to 11 using the stream recovered in step B).

FIG. 1a

FIG. 1b

FIG. 1c

FIG. 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 03068716 A
- WO 9819982 A
- WO 2012011609 A
- WO 2007079431 A
- EP 939071 A
- WO 2008054781 A
- WO 2008040969 A
- US 4902838 A
- WO 2009118628 A